(19) Europäisches Patentamt
European Patent Office
Office européen des brevets

(11) EP 4 446 365 A1

(12) EUROPEAN PATENT APPLICATION
published in accordance with Art. 153(4) EPC

(43) Date of publication:
**16.10.2024 Bulletin 2024/42**

(21) Application number: **22904136.3**

(22) Date of filing: **01.12.2022**

(51) International Patent Classification (IPC):
***C08J 3/16*** *(2006.01)* ***C08J 3/24*** *(2006.01)*
***A61F 13/53*** *(2006.01)* ***A61F 13/537*** *(2006.01)*
***B01J 20/26*** *(2006.01)* ***B01J 20/28*** *(2006.01)*

(52) Cooperative Patent Classification (CPC):
**A61F 13/53; A61F 13/537; B01J 20/26; B01J 20/28; C08J 3/16; C08J 3/24**

(86) International application number:
**PCT/JP2022/044391**

(87) International publication number:
**WO 2023/106205 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.12.2021 JP 2021198739**

(71) Applicant: **Nippon Shokubai Co., Ltd.**
**Osaka-shi, Osaka 541-0043 (JP)**

(72) Inventors:
- **TSURU, Kanako**
  **Himeji-shi, Hyogo 671-1282 (JP)**
- **MATSUMOTO, Satoshi**
  **Himeji-shi, Hyogo 671-1282 (JP)**
- **KITABATA, Sachie**
  **Himeji-shi, Hyogo 671-1282 (JP)**
- **ISHIZAKI, Kunihiko**
  **Himeji-shi, Hyogo 671-1282 (JP)**

(74) Representative: **Hoffmann Eitle**
**Patent- und Rechtsanwälte PartmbB**
**Arabellastraße 30**
**81925 München (DE)**

# (54) ABSORBENT ARTICLE

(57) Provided as an absorbent article capable of, in a state of having a crease, preventing the occurrence of liquid running along the crease is an absorbent article including a liquid-permeable top sheet, an absorbent body containing a water-absorbing resin, the water-absorbing resin being a particulate poly(meth)acrylic acid (salt)-based water-absorbing resin and having the following physical property values (1) to (3), and a liquid-impermeable back sheet in this order, wherein a basis weight of the water-absorbing resin is 300 g/m$^2$ to 1000 g/m$^2$, (1) a vertical liquid diffusion length (D) is 15 mm or more; (2) a ratio (L/D) of a horizontal liquid diffusion length (L) to the vertical liquid diffusion length is 0.5 to 1.5; and (3) a bulk density defined in EDANA ERT 460.2-02 is 0.68 g/mL or more.

FIG. 5

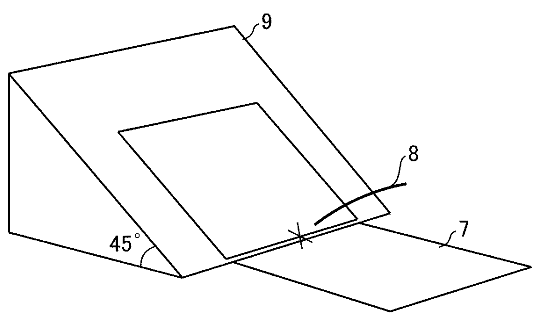


EP 4 446 365 A1

## Description

### Technical Field

The present invention relates to an absorbent article. More specifically, the present invention relates to an absorbent article that includes an absorbent body containing a poly(meth)acrylic acid (salt)-based water-absorbing resin.

### Background Art

As an absorbent article, there are known hygienic materials that absorb a body fluid, such as a disposable diaper, a sanitary napkin, and an incontinence pad. In recent years, in the hygienic materials, a water-absorbing resin as a constituent material of the hygienic materials is widely utilized as a water-absorbing agent from the viewpoint of body fluid absorption. As such a water-absorbing resin, there are known, for example, a hydrolyzate of a starch-acrylonitrile graft copolymer, a neutralized product of a starch-acrylic acid graft polymer, a saponified product of a vinyl acetate-acrylic ester copolymer, a crosslinked product of a partially neutralized (meth)acrylic acid polymer, and the like. Among them, from the viewpoint of water absorption performance, a poly(meth)acrylic acid (salt)-based water-absorbing resin in which a (meth)acrylic acid and/or a salt thereof is/are used as a main component of a monomer(s) is industrially most often produced.

As the properties demanded of such a poly(meth)acrylic acid (salt)-based water-absorbing resin, there are known multiple properties (parameters) such as a water absorption capacity without pressure (CRC), a water absorption capacity under pressure (AAP), a water absorption speed (FSR/ vortex), liquid permeability without pressure, liquid permeability under pressure, impact resistance, urine resistance, fluidity, gel strength, color, and particle size. Further, even in the same physical properties (for example, a water absorption capacity without pressure), many stipulations (parameter measurement methods) have been proposed in various viewpoints. Water-absorbing resins developed with attention focused on these many physical properties (for example, "water absorption capacity without pressure (CRC)", "water absorption capacity under pressure (AAP)", and the like), even though they are subjected to control in the above-described multiple physical properties, have a problem that it is still difficult to say that such water-absorbing resins exhibit performance sufficient to be implemented as an absorbent body such as a disposable diaper.

As a technique of exhibiting excellent performance for implementation as an absorbent body such as a disposable diaper, Patent Literature 1 discloses a method for producing a water-absorbing agent that has wettability improved by hydrophilization treatment. Further, Patent Literature 2 discloses a water-absorbing agent having excellent liquid diffusibility. Furthermore, Patent Literature 3 discloses a water-absorbing resin which has excellent water absorption speed in the initial stage and excellent liquid permeability after water absorption and in which a liquid having come into contact with an aggregate of particles of the water-absorbing resin easily permeates into and spreads on the entire aggregate. In addition, Patent Literature 4 discloses that an improvement in permeation speed at which a water-based liquid such as urine permeates through an absorbent body containing a water-absorbing resin is achieved by controlling, within a specific range, the diffusibility of a physiological saline in a swollen gel layer which is obtained by allowing the water-absorbing resin to absorb water under a specific condition and is then allowed to absorb a large volume of physiological saline for a plurality of times.

### Citation List

[Patent Literature]

[Patent Literature 1]
Japanese Patent Application Publication Tokukaihei No. 11-71425
[Patent Literature 2]
Publication of Japanese Patent No. 02918807
[Patent Literature 3]
Pamphlet of International Publication No. WO 2020/122215
[Patent Literature 4]
Pamphlet of International Publication No. WO 2021/049450

## Summary of Invention

### Technical Problem

An absorbent article such as a diaper can be packaged in a folded state. Thus, an absorbent article packaged in a folded state has a line produced by folding, that is, a crease. In addition, even during wearing of an absorbent article, if the absorbent article is not in close contact with the body, a crease and/or a wrinkle may be formed on the absorbent article.

In a case where an absorbent article containing the conventional water-absorbing resin is allowed to absorb a water-based liquid such as urine in a state in which the absorbent article has a crease, the water-based liquid is highly likely to be leaked from sides of the absorbent article due to the occurrence of liquid running which is diffusion of the liquid on a surface of the absorbent article and a surface layer part thereof along the crease toward the sides of the absorbent article, that is, end portions of the absorbent article.

An object of the present invention is to reduce or prevent leakage of the water-based liquid from the sides of an absorbent article due to the occurrence of liquid running along the above-described crease.

### Solution to Problem

The inventors of the present invention have found that suitable as an absorbent article capable of reducing or preventing leakage of a liquid from sides of the absorbent article which has been allowed to absorb the liquid due to the occurrence of liquid running along the above-described crease is an absorbent article which contains a poly(meth)acrylic acid (salt)-based water-absorbing resin having the following physical property values (1) to (3) and in which a basis weight of the water-absorbing resin is controlled within a specific range. The inventors have thus finally completed the present invention.

An absorbent article in accordance with an embodiment of the present invention is an absorbent article including a liquid-permeable top sheet, an absorbent body containing a water-absorbing resin, and a liquid-impermeable back sheet in this order, wherein

a basis weight of the water-absorbing resin is 300 g/m$^2$ to 1000 g/m$^2$, and
the water-absorbing resin is a particulate poly(meth)acrylic acid (salt)-based water-absorbing resin and has the following physical property values (1) to (3):

(1) a vertical liquid diffusion length (D) is 15 mm or more;
(2) a ratio (L/D) of a horizontal liquid diffusion length (L) to the vertical liquid diffusion length is 0.5 to 1.5; and
(3) a bulk density defined in EDANA ERT 460.2-02 is 0.68 g/mL or more,
where the vertical liquid diffusion length (D) and the horizontal liquid diffusion length (L) are determined by filling a transparent container made of an acrylic resin (having internal dimensions of 40 mm in a vertical direction (height) by 72 mm in a horizontal direction (width) by 3 mm in depth) with the water-absorbing resin up to a position at a height of 30 mm, injecting, from a surface of the water-absorbing resin, 1 mL of a 0.9 mass% aqueous sodium chloride solution which is colored in blue and has a temperature of 20°C, and measuring a diffusion length in the vertical direction and a diffusion length in the horizontal direction 1 minute after the injection.

### Advantageous Effects of Invention

According to an absorbent article in accordance with the present invention, in a case where the absorbent article is allowed to absorb a water-based liquid such as urine in a state in which the absorbent article has a crease, it is possible to reduce or prevent leakage of the water-based liquid from the sides of the absorbent article due to the occurrence of liquid running along the crease.

### Brief Description of Drawings

Fig. 1 is a perspective view illustrating a container for measuring liquid diffusion lengths of a water-absorbing resin.
Fig. 2 is a schematic view for describing the measurement of the liquid diffusion lengths of a water-absorbing resin.
Fig. 3 is a view illustrating an absorbent sheet for evaluation of the amount of liquid return from a water-absorbing

resin as viewed from directly above.

Fig. 4 is a cross-sectional view illustrating the absorbent sheet for evaluation of the amount of liquid return from a water-absorbing resin.

Fig. 5 is a schematic view (perspective view) for describing a method for measuring liquid diffusion lengths of an absorbent article in the longitudinal, lateral, upward, and downward directions.

Fig. 6 is a schematic view (elevational view) for describing the method for measuring liquid diffusion lengths of an absorbent article in the longitudinal, lateral, upward, and downward directions.

Description of Embodiments

The following description will discuss the present invention based on the best mode of the present invention. Any term used in the present specification should be understood as ordinarily used in this technical field unless particularly mentioned otherwise. Therefore, unless defined otherwise, all of the technical terms and scientific terms used in the present specification mean as generally understood by a person skilled in the technical field to which the present invention belongs. If there is any conflict in meaning, the present specification (including the definitions) take priority. The present invention is not limited to the embodiments described below and can be altered in various ways within the scope of the patent claims.

An absorbent article in accordance with an embodiment of the present invention is an absorbent article including a liquid-permeable top sheet, an absorbent body containing a water-absorbing resin, and a liquid-impermeable back sheet in this order, wherein

a basis weight of the water-absorbing resin is 300 g/m$^2$ to 1000 g/m$^2$, and

the water-absorbing resin is a particulate poly(meth)acrylic acid (salt)-based water-absorbing resin and has the following physical property values (1) to (3):

(1) a vertical liquid diffusion length (D) is 15 mm or more;

(2) a ratio (L/D) of a horizontal liquid diffusion length (L) to the vertical liquid diffusion length is 0.5 to 1.5; and

(3) a bulk density defined in EDANA ERT 460.2-02 is 0.68 g/mL or more,

where the vertical liquid diffusion length (D) and the horizontal liquid diffusion length (L) are determined by filling a transparent container made of an acrylic resin (having internal dimensions of 40 mm in a vertical direction (height) by 72 mm in a horizontal direction (width) by 3 mm in depth) with the water-absorbing resin up to a position at a height of 30 mm, injecting, from a surface of the water-absorbing resin, 1 mL of a 0.9 mass% aqueous sodium chloride solution colored in blue and having a temperature of 20°C, and measuring a diffusion length in the vertical direction and a diffusion length in the horizontal direction 1 minute after the injection.

In the absorbent article in the present invention, a mechanism capable of preventing leakage from a crease can be considered, for example, as below.

It is considered that a high basis weight of a water-absorbing resin prevents an absorbent article from having a region in which the water-absorbing resin does not exist in a crease of the absorbent article. In addition, a high bulk density of a water-absorbing resin enables a reduction in size of a gap between particles of the water-absorbing resin generated in a crease portion of an absorbent article. As a result, liquid running does not occur in the crease portion of the absorbent article. Moreover, the water-absorbing resin used in the present invention exhibits good liquid permeability and diffusibility even in a state of being densely packed. Thus, a liquid is uniformly diffused in a water-absorbing resin layer without a risk of leakage.

<1> Definitions of terms

< 1-1> Water-absorbing resin

The term "water-absorbing resin" as used in the present invention refers to a water-swellable and water-insoluble polymer gelling agent that satisfies the following physical properties. Specifically, the term "water-absorbing resin" refers to a polymer gelling agent that satisfies the following physical properties: CRC (centrifuge retention capacity) defined in ERT 441.2-02 as "water-swelling property" is 5 g/g or more, and Ext (extractable) defined in ERT 470.2-02 as "water-insolubility" is 50 weight% or less.

The water-absorbing resin can be designed as appropriate according to its purpose of use, and is not limited to a particular design. The water-absorbing resin is preferably a hydrophilic crosslinked polymer that has been obtained

by crosslinking and polymerizing unsaturated monomers each of which has a carboxyl group. In addition, the water-absorbing resin is not limited to a form in which the water-absorbing resin is wholly (100 weight%) a polymer, and can be a water-absorbing resin composition containing an additive and the like within a range in which the above-described physical properties (CRC and Ext) are satisfied. The water-absorbing resin in the present invention may refer to not only an end product but also an intermediate produced during a process of producing the water-absorbing resin (e.g. a crosslinked hydrogel polymer after polymerization, a dried polymer after drying, a water-absorbing resin powder before surface crosslinking, and the like). All these water-absorbing resins, including the water-absorbing resin composition described above, will be collectively referred to as "water-absorbing resin". Examples of forms of the water-absorbing resin encompass a sheet form, a fiber form, a film form, and a particulate form. Among them, the water-absorbing resin in the present invention is preferably a particulate water-absorbing resin.

<1-2> "EDANA" and "ERT"

The term "EDANA" is an acronym for the European Disposables and Nonwovens Associations. The term "ERT" is an acronym for EDANA Recommended Test Methods, which are European standard (de facto international standard) measuring methods for a water-absorbing resin. For the present invention, physical properties of a water-absorbing resin are measured in conformity with the ERT master copy (2002 revised version; known literature) unless otherwise specified.

<1-3> Others

In the present specification, any range of "X to Y" means "X or more and Y or less".

In the present specification, "ppm" means "mass ppm" unless otherwise specified.

In the present specification, the term "... acid (salt)" means "... acid and/or a salt thereof". The term "(meth)acrylic" means "acrylic and/or methacrylic". The term "poly(meth)acrylic acid (salt)-based water-absorbing resin" means a water-absorbing resin that contains, as a main component, a repeating unit of (meth)acrylic acid (salt), and specifically refers to a water-absorbing resin containing (meth)acrylic acid (salt) that accounts for preferably 50 mol% to 100 mol%, more preferably 70 mol% to 100 mol%, even more preferably 90 mol% to 100 mol%, and particularly preferably substantially 100 mol% of all monomers to be used for polymerization (excluding a crosslinking agent).

In the present specification, the unit of volume "liter" may be denoted as "1" or "L".

In the present specification, the terms "weight" and "mass" are used synonymously, the terms "weight%" and "mass%" are used synonymously, and the terms "parts by weight" and "parts by mass" are used synonymously.

<2> Water-absorbing resin

<2-1> Shape of water-absorbing resin

A water-absorbing resin (hereinafter also referred to as "water-absorbing resin in the present invention") contained in an absorbent article in accordance with an embodiment of the present invention is preferably in the form of particles, and specific examples of the shape of the particles encompass a non-uniformly pulverized shape, a spherical shape, a football shape, and an aggregate shape, and the like. Above all, a water-absorbing resin in the form of particles having a spherical shape has an increased bulk density, and a water-absorbing resin in the form of particles having an aggregate shape has an improved water absorption speed. Thus, the water-absorbing resin is preferably an aggregate-like particle of primary particles (spherical particles). Note here that the spherical shape encompasses not only a true spherical shape but also a substantially spherical shape of an aspect ratio of 1.0 to 1.2.

<2-2> Additive to be contained

In the present invention, an additive for allowing a water-absorbing resin to exhibit various functions can be contained. Specifically, examples of such an additive encompass a surfactant, a compound having a phosphorus atom, an oxidizer, an organic reducing agent, water-insoluble inorganic fine particles, organic powder such as metallic soap, a deodorizing agent, an antibacterial agent, pulp, thermoplastic fibers, and the like. Note that, as the water-insoluble inorganic fine particles, a compound disclosed in "<5> Water-insoluble inorganic fine particles" of International Publication No. WO 2011/040530 is employed in the present invention. Among these water-insoluble inorganic fine particles, hydrophilic fine particles in particular (fine particles having a high degree (for example, 70% or more) of hydrophilicity (expressed by a proportion of fine particles suspended in the form of a colloid in a mixed solution of water and methanol in a 70:30 ratio) described in European Patent No. 0629411 and fine particles having a small contact angle (for example, 10 degrees or less) with respect to water described in Japanese Patent No. 6837139), for example, at least one selected

from the group consisting of silica (silicon dioxide) and hydrotalcite, are preferably contained because a resulting water-absorbing resin (for example, water-absorbing resin particles) has an improved compatibility with a liquid, and the water-absorbing resin, when used in an absorbent article, can absorb a water-based liquid in a short time.

From the viewpoint of improving compatibility of the water-absorbing resin (for example, water-absorbing resin particles) with a liquid, an amount of the water-insoluble inorganic fine particles contained is 0.01 parts by mass to 5 parts by mass, more preferably 0.05 parts by mass to 3 parts by mass, and even more preferably 0.1 parts by mass to 1 part by mass, and particularly preferably 0.2 parts by mass to 0.5 parts by mass, relative to 100 parts by mass of the water-absorbing resin.

The water-insoluble inorganic fine particles herein ordinarily have a minute size compared to the size of polymer particles. For example, the average particle diameter of the water-insoluble inorganic fine particles may be 0.01 pm to 50 pm, 0.1 pm to 30 pm, or 1 pm to 20 pm. The average particle diameter can be measured by a pore electric resistance method or a laser diffraction and scattering method according to the properties of particles.

<2-3> CRC

The term "CRC" is an acronym for centrifuge retention capacity and means a water absorption capacity without pressure of a water-absorbing resin. In the present specification, the CRC is measured in conformity with an EDANA method (ERT 441.2-02).

The CRC of the water-absorbing resin in the present invention is preferably 30 g/g or more, and more preferably 32 g/g or more. The upper limit of the CRC is not particularly limited, and a higher CRC is preferable. From the viewpoint of a balance with other physical properties, the CRC is preferably 50 g/g or less, and more preferably 48 g/g or less, 46 g/g or less, 44 g/g or less, 42 g/g or less, 40 g/g or less, 38 g/g or less, and 36 g/g or less.

A water-absorbing resin having a CRC of 30 g/g or more achieves a sufficient absorption amount and is thus suitable as an absorbent body for an absorbent article such as a disposable diaper. Further, a water-absorbing resin having a CRC of 50 g/g or less prevents a decrease in the speed at which a body fluid or the like such as urine and blood is absorbed and is suitable for use in, for example, a disposable diaper having a high water absorption speed. Note that the value of the CRC can be controlled by changing the type and/or amount of, for example, an internal crosslinking agent or a surface-crosslinking agent.

<2-4> Ext

The term "Ext" is an abbreviation for "Extractables" (water-soluble component) and means an amount of water-soluble component extracted from a water-absorbing resin. The water-soluble component is measured in conformity with an EDANA method (ERT 470.2-02).

The Ext of the water-absorbing resin in the present invention is preferably 30 mass% or less, more preferably 20 mass% or less, and even more preferably 10 mass% or less. The lower limit of the Ext is 0 mass%. From the viewpoint of a balance with other physical properties, the lower limit of the Ext is preferably 2 mass% or more, and more preferably 4 mass% or more.

A water-absorbing resin having an Ext of 30 mass% or less prevents a decrease in the speed at which a body fluid or the like such as urine and blood is absorbed and is suitable for use in, for example, a disposable diaper having a high water absorption speed. Note that the value of the Ext can be controlled by changing the type and/or amount of, for example, a polymerization initiator, an internal crosslinking agent, or a surface-crosslinking agent, and can also be controlled by using a chain transfer agent in the polymerization step.

<2-5> AAP

The term "AAP" is an acronym for "absorption against pressure", and means a water absorption capacity under pressure of a water-absorbing resin. In the present invention, the AAP is measured in conformity with an EDANA method (ERT 442.2-02) except that a load condition is changed to 4.83 kPa (0.7 psi). Specifically, the AAP (absorption capacity under pressure) (unit: g/g) is measured after 0.9 g of water-absorbing resin has been allowed to swell for 1 hour under a load of 4.83 kPa with use of a 0.9 mass% aqueous sodium chloride solution.

The AAP of the water-absorbing resin in the present invention is preferably 18 g/g or more, more preferably 20 g/g or more, and even more preferably 23 g/g or more, from the viewpoint of water absorption property when used in hygienic materials. Further, the upper limit of the AAP of the water-absorbing resin is not particularly limited, and is preferably 40 g/g or less.

<2-6> Moisture content

The "moisture content" is measured in conformity with an EDANA method (ERT 430.2-02), except that the amount of a sample is changed to 1.0 g, and a drying temperature is changed to 180°C.

The moisture content of the water-absorbing resin in the present invention is not particularly limited, and is preferably 1 mass% to 20 mass%, more preferably 1 mass% to 15 mass%, even more preferably 2 mass% to 13 mass%, still more preferably 5 mass% to 13 mass%, further more preferably 8 mass% to 13 mass%, and particularly preferably 10 mass% to 13 mass%. A water-absorbing resin having a moisture content of 1 mass% to 20 mass% prevents a decrease in the speed at which a body fluid or the like such as urine and blood is absorbed and is suitable for use in, for example, a disposable diaper having a high water absorption speed.

<2-7> Mass average particle diameter (D50)

The "mass average particle diameter (D50)" is measured in conformity with "(3) Mass-Average Particle Diameter (D50) and Logarithmic Standard Deviation (σζ) of Particle Diameter Distribution" described in Columns 27 and 28 of US Patent No. 7,638,570.

The mass average particle diameter (D50) of the water-absorbing resin in the present invention is preferably 50 pm to 800 pm, more preferably 200 pm to 700 pm, even more preferably 250 pm to 600 pm, and particularly preferably 250 pm to 500 pm. Further, the proportion of particles having a particle diameter of less than 45 pm is preferably 40 mass% or less, more preferably 30 mass% or less, even more preferably 20 mass% or less, and particularly preferably 10 mass% or less. A water-absorbing having a mass average particle diameter of 200 pm or more generates less dust and provides good handleability. Further, a water-absorbing resin having a mass average particle diameter of 700 pm or less prevents a decrease in the speed at which a body fluid or the like such as urine and blood is absorbed and is suitable for use in, for example, a disposable diaper having a high water absorption speed.

<2-8> Number average particle diameter

In a case where a water-absorbing resin is an aggregate-like particle of aggregated primary particles, the number average particle diameter of the primary particles constituting the aggregate is measured with use of an electron microscope. The number average particle diameter of the primary particles of the water-absorbing resin is preferably 5 pm to 800 pm, more preferably 8 pm to 500 pm, even more preferably 10 pm to 300 pm, still more preferably 10 pm to 200 pm, and particularly preferably 30 pm to 100 pm.

<2-9> Surface tension

The "surface tension" in the present invention is a surface tension of an aqueous solution obtained by dispersing a water-absorbing resin in a 0.9 mass% aqueous sodium chloride solution, and is measured by a method described in WO 2015/129917.

The surface tension of the water-absorbing resin in the present invention may be 74 mN/m or less, may be 55 mN/m or more, or is preferably 60 mN/m or more, more preferably 65 mN/m or more, and even more preferably 70 mN/m or more. The surface tension falling within the above-described range is preferable since liquid compatibility with a water-based liquid is excellent.

<2-10> Vertical liquid diffusion length (D)

The "vertical liquid diffusion length" in the present invention is a novel physical property value for evaluating the extent to which a water-based liquid is diffused in a water-absorbing resin layer in a vertical direction thereof, and is specifically a value measured by a measurement method which is described in Examples. The vertical liquid diffusion length is measured by a method described later. A water-absorbing resin having a long vertical liquid diffusion length represents that the water-absorbing resin has good liquid compatibility with a water-based liquid added to the water-absorbing resin. A water-absorbing resin having good liquid compatibility quickly takes in a liquid in, for example, a thin water-absorbing sheet for light incontinence or the like application and diffuses the liquid in the water-absorbing sheet, and thus achieves a reduction in amount of liquid return immediately after liquid absorption. In addition, a water-absorbing resin having good liquid compatibility, when a water-based liquid such as urine is added to an absorbent article containing the water-absorbing resin and being in a state of having a crease, quickly takes in the added water-based liquid inside the absorbent article and thus makes the liquid less likely to flow along the crease and on the surface of the absorbent article and the surface layer part thereof. As a result, it is possible to suitably reduce or prevent the occurrence of liquid running along the crease. From the viewpoint of suitably reducing or preventing leakage of an added water-based liquid

from sides of an absorbent article due to the occurrence of the liquid running along the crease, the vertical liquid diffusion length of the water-absorbing resin in the present invention is 15 mm or more, preferably 16 mm or more, more preferably 17 mm or more, even more preferably 18 mm or more, particularly preferably 19 mm or more, and extremely preferably 20 mm or more. In the water-absorbing resin in the present invention, the upper limit of the vertical liquid diffusion length is 30 mm, which is the height of a resin layer in the measurement, or less. A preferable range of the upper limit of the vertical liquid diffusion length is not particularly limited and may be 28 mm or less and 25 mm or less.

<2-11> Horizontal liquid diffusion length (L)

The "horizontal liquid diffusion length" in the present invention is a novel physical property value that represents the extent to which a water-based liquid is diffused in a water-absorbing resin layer in a horizontal direction thereof, and is specifically a value measured by a measurement method which is described in Examples. The horizontal liquid diffusion length is measured by a method described later. A water-absorbing resin having a long horizontal liquid diffusion length represents that the water-absorbing resin has poor liquid compatibility with a water-based liquid added to the water-absorbing resin and makes liquid running more likely to occur in the horizontal direction. A water-absorbing resin having poor liquid compatibility is not preferable for the reason that there is a possibility that leakage may occur because a water-based liquid is not quickly absorbed in an absorbent body and an absorbent article that includes the absorbent body, and liquid running occurs on the surface of the absorbent article and the surface layer part of the absorbent article such as the surface of the absorbent body. The horizontal liquid diffusion length of the water-absorbing resin in the present invention is 72 mm, which is a width of a resin layer in the measurement, or less, preferably 35 mm or less, even more preferably 30 mm or less, and particularly preferably 25 mm. The lower limit of the horizontal liquid diffusion length of the water-absorbing resin in the present invention is not particularly limited and may be 10 mm or more and 15 mm or more.

<2-12> L/D

The "L/D" in the present invention represents a ratio of the "vertical liquid diffusion length (D)" to the above-described "horizontal liquid diffusion length (L)". A water-absorbing resin having a ratio of L/D closer to 1 uniformly diffuses a water-based liquid in a water-absorbing resin layer. Thus, such a water-absorbing resin, when used in an absorbent body, enables a water-based liquid to be diffused in the absorbent body without imbalance and enables a reduction in amount of liquid return immediately after liquid absorption.

The L/D is in the range of 0.5 to 1.5, preferably in the range of 0.6 to 1.5, more preferably in the range of 0.7 to 1.4, particularly preferably in the range of 0.8 to 1.4, and extremely preferably in the range of 0.9 to 1.4.

<2-13> Bulk density

The bulk density is a mass (unit: [g/mL]) of a water-absorbing resin when 100 g of a water-absorbing resin is charged into a device defined by an EDANA method (ERT 460.2-02), and the water-absorbing resin is freely dropped into a 100 mL container to fill the container. A water-absorbing resin having a high bulk density is densely filled in, for example, a thin water-absorbing sheet for light incontinence or the like application, and a gap is less likely to be generated between particles of the water-absorbing resin. When a gap is generated between particles of a water-absorbing resin, a water-based liquid enters the gap and remains without being absorbed by the water-absorbing resin. This leads to an increase in amount of liquid return immediately after liquid absorption. For this reason, the generation of a gap between particles of a water-absorbing resin is not preferable. In addition, in a case where a gap is generated between particles of a water-absorbing resin, it is considered that there is a possibility that liquid running may occur. This is because, when a water-based liquid such as urine is added to an absorbent article containing the water-absorbing resin and being in a state of having a crease, a remaining water-based liquid which remains unabsorbed on a surface of the absorbent article and a surface layer part thereof moves along the crease and on the surface of the absorbent article. Therefore, a water-absorbing resin preferably has a high bulk density from the viewpoint of suitably reducing or preventing leakage of an added water-based liquid from sides of an absorbent article due to the occurrence of the liquid running along the crease. Specifically, in the water-absorbing resin in the present invention, the bulk density is 0.68 g/mL or more, preferably 0.69 g/mL or more, and particularly preferably 0.70 g/mL or more. In the water-absorbing resin in the present invention, the upper limit of the bulk density is not particularly limited and may be 1.00 g/mL or less, 0.95 g/mL or less, and 0.90 g/mL or less.

<3> Method for producing water-absorbing resin

A method for producing the water-absorbing resin in the present invention may use any of the following: aqueous

solution polymerization, reversed phase suspension polymerization, vapor phase droplet polymerization, and other polymerization methods. The above-described production method will be described below by taking reversed phase suspension polymerization as an example from the viewpoint of ease of control of the physical properties of the water-absorbing resin in the present invention. In particular, the above-described production method will be described by taking, as an example, a production method including a step of separating a gel obtained by reversed phase suspension polymerization, a gel sizing step, a drying step of drying a hydrogel (preferably hot air drying), and a surface-crosslinking step (preferably powder surface treatment), unlike general reversed phase suspension polymerization including an azeotropic dehydration step in a hydrophobic organic solvent after polymerization and/or a surface-crosslinking step in a dispersion system.

The method for producing the water-absorbing resin in accordance with an embodiment of the present invention includes a polymerization step of obtaining a hydrogel polymer (water-containing gel polymer) by, in a state in which droplets containing a monomer are dispersed or suspended in a hydrophobic organic solvent, polymerizing the monomer.

The polymerization method only needs to be a polymerization method of obtaining a hydrogel polymer by reversed phase suspension polymerization in which a hydrogel polymer is obtained by polymerizing a monomer in a state in which droplets containing the monomer are dispersed or suspended in a liquid phase composed of a hydrophobic organic solvent. Such a polymerization method may be a batch-type method or a continuous-type method. A batch-type production method is a production method in which an aqueous monomer solution is added or dropped into a hydrophobic organic solvent in a reaction apparatus and mixed to disperse or suspend the aqueous monomer solution, and then polymerization is carried out to obtain a hydrogel polymer. On the other hand, a continuous-type production method is a method in which an aqueous monomer solution is continuously fed to a hydrophobic organic solvent in a reaction apparatus, dispersed or suspended, and then polymerized, and a hydrogel polymer formed by a polymerization reaction and the hydrophobic organic solvent are continuously discharged from the reaction apparatus. A preferable embodiment of the present invention is batch-type reversed phase suspension polymerization. In the method for producing the water-absorbing resin in accordance with the present invention, a separation step of separating the hydrogel polymer obtained in the polymerization step from the hydrophobic organic solvent may be provided.

The method for producing the water-absorbing resin in accordance with an embodiment of the present invention includes: any aqueous monomer solution preparation step; any dispersion step; a polymerization step; any separation step; any gel sizing step; a drying step; and a hydrophilization treatment step. Further, the method can optionally include, after the drying step, a cooling step, a pulverizing step, a classification step, a surface-crosslinking step, a hydration (re-moistening) step, an other additive addition step, a sizing step, a fine powder removal step, a granulation step, a fine powder reuse step, and the like. In addition, the method may further include a conveying step, a storing step, a packing step, a reserving step, and the like.

The individual steps will be described below.

<3-1> Aqueous monomer solution preparation step

The aqueous monomer solution is an aqueous solution that contains a monomer which serves as a raw material for a water-absorbing resin, and is a solution to be dispersed or suspended in a hydrophobic organic solvent in order to carry out reversed phase suspension polymerization.

As the solvent of the aqueous monomer solution, water or a mixture of water and a water-soluble organic solvent (for example, alcohol or the like) is suitably used. The solvent of the aqueous monomer solution is even more preferably water. In a case where the solvent is a mixture of water and a water-soluble organic solvent, the water-soluble organic solvent (for example, alcohol or the like) accounts for preferably 30 mass% or less and more preferably 5 mass% or less of the mixture.

As the monomer, (meth)acrylic acid which is a water-soluble ethylenically unsaturated monomer is used. The monomer is particularly preferably acrylic acid.

Note that a polymerization inhibitor may be added, as necessary, in consideration of stability of the (meth)acrylic acid.

In a case where an acid group-containing unsaturated monomer is used as the monomer, the acid group-containing unsaturated monomer and a neutralized salt of the acid group-containing unsaturated monomer are preferably used in combination from the viewpoint of water absorption performance of a resulting water-absorbing resin. From the viewpoint of water absorption performance, the number of moles of the neutralized salt relative to the total number of moles of the acid group-containing unsaturated monomer and the neutralized salt thereof (hereinafter, referred to as "neutralization ratio") is preferably 40 mol% or more, more preferably 40 mol% to 95 mol%, further preferably 50 mol% to 90 mol%, even further preferably 55 mol% to 85 mol%, and particularly preferably 60 mol% to 80 mol%.

In the production method in accordance with the present invention, in the preparation of the aqueous monomer solution, any of the monomers listed above as examples may be used solely, or any two or more of the monomers may be mixed as appropriate and used. In addition, another monomer may be further mixed and used, provided that the

object of the present invention is achieved. In a case where two or more of the monomers are used in combination in the preparation of the aqueous monomer solution, the monomers preferably include (meth)acrylic acid (salt) as a main component. In this case, from the viewpoint of the water absorption performance of a resulting water-absorbing resin, the proportion of the (meth)acrylic acid (salt) to the entire monomer used for the polymerization is ordinarily 50 mol% or more, preferably 70 mol% or more, more preferably 80 mol% or more, and further preferably 90 mol% or more (the upper limit is 100 mol%).

In the preparation of the aqueous monomer solution, an internal crosslinking agent can be used as necessary. The internal crosslinking agent is exemplified by a conventionally known internal crosslinking agent having two or more polymerizable unsaturated groups or two or more reactive groups within one molecule thereof. Examples of the internal crosslinking agent encompass N,N'-methylene bis(meth)acrylamide, (poly)ethylene glycol di(meth)acrylate, (poly)propylene glycol di(meth)acrylate, trimethylol propane tri(meth)acrylate, trimethylol propane di(meth)acrylate, glycerin tri(meth)acrylate, glycerin acrylate methacrylate, ethylene oxide-modified trimethylol propane tri(meth)acrylate, pentaerythritol tetra(meth)acrylate, dipentaerythritol hexa(meth)acrylate, triallyl cyanurate, triallyl isocyanurate, triallyl phosphate, triallylamine, poly(meth)allyloxy alkane, (poly)ethylene glycol diglycidyl ether, glycerol diglycidyl ether, ethylene glycol, polyethylene glycol, propylene glycol, glycerin, 1,4-butanediol, pentaerythritol, ethylenediamine, ethylene carbonate, propylene carbonate, polyethyleneimine, glycidyl (meth)acrylate, and the like. Only one of these internal crosslinking agents may be used, or two or more thereof may be used.

The amount of the internal crosslinking agent used is determined as appropriate in accordance with the physical properties of a desired water-absorbing resin and is ordinarily 0.0001 mol% to 5 mol%, more preferably 0.001 mol% to 3 mol%, and even more preferably 0.005 mol% to 1.5 mol%, relative to the monomer.

In addition, substances (hereinafter, referred to as "other substances") examples of which will be listed below can also be added to the aqueous monomer solution.

Specific examples of the other substances encompass: chain transfer agents such as thiols, thiolic acids, secondary alcohols, amines, and hypophosphites; foaming agents such as carbonates, bicarbonates, azo compounds, and bubbles; chelating agents such as metal salts of ethylenediamine tetraacetic acid, and metal salts of diethylenetriamine pentaacetic acid; polyacrylic acid (salt) and crosslinked products thereof; starch; cellulose; starch-cellulose derivatives; polyvinyl alcohol; and the like. The other substances may be used solely, or two or more of the other substances may be used in combination.

The amount of the other substances used is not particularly limited. The total concentration of the other substances in the aqueous monomer solution is preferably 10 mass% or less, more preferably 1 mass% or less, and even more preferably 0.1 mass% or less, relative to the monomer. However, the total concentration of polyacrylic acid (salt) and crosslinked products thereof, starch, cellulose, starch-cellulose derivatives, or polyvinyl alcohol in the aqueous monomer solution is preferably 30 mass% or less, more preferably 20 mass% or less, and even more preferably 10 mass% or less, relative to the monomer.

Further, dissolved oxygen in the aqueous monomer solution may be reduced by temperature increase or substitution with an inert gas.

"Polymerization initiator"

In the preparation of the aqueous monomer solution, a polymerization initiator may be used. Note that, in a case where a polymerization initiator is used in the preparation of an aqueous monomer solution, gelation of the aqueous monomer solution and/or increase in viscosity thereof may occur. Thus, preferably carried out are, for example, the following operations: the addition of the polymerization initiator is carried out immediately before the aqueous monomer solution is dispersed or suspended in a hydrophobic organic solvent; the aqueous monomer solution is cooled and is mixed with the polymerization initiator at a temperature (20°C or lower, preferably around 0°C) lower than normal temperature; and the dispersion step is carried out while the aqueous monomer solution and the polymerization initiator are line-mixed. As the polymerization initiator, a pyrolytic polymerization initiator is preferably used. The pyrolytic polymerization initiator refers to a compound that is decomposed by heat to generate radicals. From the viewpoint of storage stability of the pyrolytic polymerization initiator and/or production efficiency of a water-absorbing resin, preferably used as the polymerization initiator is a water-soluble compound having a 10-hour half-life temperature of preferably 0°C to 120°C, more preferably 30°C to 100°C, and further preferably 50°C to 80°C.

From the viewpoint of handleability and/or physical properties of a water-absorbing resin, the polymerization initiator is preferably a water-soluble polymerization initiator, and more preferably a water-soluble radical polymerization initiator.

Examples of the water-soluble radical polymerization initiator encompass: persulfates such as potassium persulfate, ammonium persulfate, and sodium persulfate; peroxides such as methyl ethyl ketone peroxide, methyl isobutyl ketone peroxide, di-t-butyl peroxide, t-butyl cumyl peroxide, t-butylperoxyacetate, t-butylperoxyisobutyrate, t-butylperoxypivalate, and hydrogen peroxide; water-soluble azo compounds such as 2,2'-azobis(2-amidinopropane) dihydrochloride, 2,2'-azobis[2-(N-phenylamidino)propane] dihydrochloride, and 2,2'-azobis[2-(2-imidazolin-2-yl)propane] dihydrochloride; and the like. As the polymerization initiator, one of these polymerization initiators may be used solely, or two or more thereof may be used in combination. Among these polymerization initiators, persulfates or water-soluble azo compounds are preferably used, sodium persulfate, potassium persulfate, ammonium persulfate, or 2,2'-azobis(2-amidinopropane) dihydrochloride is more preferably used, and sodium persulfate is even more preferably used.

The amount of the pyrolytic polymerization initiator used is set as appropriate in accordance with, for example, the types of the monomer and of the polymerization initiator and is not particularly limited. From the viewpoint of production efficiency, the amount of the pyrolytic polymerization initiator used is preferably 0.001 g/mol or more, more preferably 0.005 g/mol or more, and even more preferably 0.01 g/mol or more, relative to the monomer. Further, from the viewpoint of improvement in water absorption performance of a water-absorbing resin, the amount of the pyrolytic polymerization initiator used is preferably 2 g/mol or less and more preferably 1 g/mol or less.

In addition, if necessary, the pyrolytic polymerization initiator can be used in combination with another polymerization initiator such as a photolytic polymerization initiator. Specific examples of the photolytic polymerization initiator encompass benzoin derivatives, benzyl derivatives, acetophenone derivatives, benzophenone derivatives, and the like.

In addition, the pyrolytic polymerization initiator and a reducing agent can be used in combination as a redox polymerization initiator. In the redox polymerization initiator, the pyrolytic polymerization initiator functions as an oxidizing agent. The reducing agent to be used in combination with the pyrolytic polymerization initiator is not particularly limited, and examples of the reducing agent encompass: (bi)sulfites such as sodium sulfite and sodium hydrogen sulfite; reducing metal salts such as a ferrous salt; L-ascorbic acid (salt); amines; and the like.

"Concentration of monomer in aqueous monomer solution"

In the present invention, the concentration of a monomer in an aqueous monomer solution is selected according to, for example, the types of a selected monomer and of a selected hydrophobic organic solvent. In terms of production efficiency, the lower limit of the concentration of the monomer in the aqueous monomer solution is preferably 10 mass% or more, more preferably 20 mass% or more, and even more preferably 30 mass% or more in the aqueous monomer solution, and the upper limit of the concentration of the monomer in the aqueous monomer solution is preferably 100 mass% or less, more preferably 90 mass% or less, even more preferably 80 mass% or less, and further more preferably 70 mass% or less.

It is also possible to blend an additive such as an internal crosslinking agent, a surfactant, a density adjusting agent, a thickener, and a chelating agent into the aqueous monomer solution, provided that the object of the present invention is not impaired. Note that the type of the additive and the amount of the additive added can be selected as appropriate depending on a combination of the monomer used and the hydrophobic organic solvent used.

<3-2> Dispersion step

The dispersion step is a step of dispersing or suspending droplets containing a monomer in a hydrophobic organic solvent. Note that, hereinafter, in a case where the term "dispersion" is simply described, the dispersion conceptually encompasses suspension. More specifically, the aqueous monomer solution is added to a hydrophobic organic solvent, mixed, and stirred so that the droplets containing the monomer are dispersed. For example, a stirring apparatus provided with a stirring blade (such as a propeller blade, a paddle blade, an anchor blade, a turbine blade, a Phaudler blade, a ribbon blade, and a flat plate blade) may be used. In a case where a stirring apparatus having such a stirring blade is used, a dispersed droplet diameter can be adjusted depending on the type of the stirring blade, a blade diameter, the number of rotations, and the like. The stirring apparatus having such a stirring blade can be particularly suitably used when batch-type reversed phase suspension polymerization is carried out. Further, a dispersion can be obtained by a method described in, for example, International Publication No. WO 2009/025235 and International Publication No. WO 2013/018571. In a case where the continuous-type reversed phase suspension polymerization is carried out, the dispersion step is preferably such that the aqueous monomer solution and the hydrophobic organic solvent are continuously and separately supplied to the dispersion apparatus, and droplets containing the monomer and dispersed in the hydrophobic organic solvent are prepared.

In a case where the continuous-type reversed phase suspension polymerization is carried out, examples of the dispersion apparatus used in the dispersion step encompass: a spray nozzle; a high-speed rotary shear type stirrer (such as a rotary mixer type, a turbo mixer type, a disk type, and a double cylinder type); a cylindrical nozzle such as a needle; an orifice plate in which a large number of holes are directly provided in the plate; a spray nozzle; a centrifugal atomizer such as a rotary wheel; and the like. However, there is no particular limitation.

"Hydrophobic organic solvent"

A preferable hydrophobic organic solvent is exemplified by at least one organic solvent selected from the group consisting of aliphatic hydrocarbons, alicyclic hydrocarbons, aromatic hydrocarbons, and halogenated hydrocarbons. Specific examples of the hydrophobic organic solvent encompass: aliphatic hydrocarbons such as n-pentane, n-hexane, n-heptane, and n-octane; alicyclic hydrocarbons such as cyclohexane, methylcyclohexane, cyclooctane, and decalin; aromatic hydrocarbons such as benzene, toluene, and xylene; and halogenated hydrocarbons such as chlorobenzene, bromobenzene, carbon tetrachloride, and 1,2-dichloroethane. Among these hydrophobic organic solvents, from the viewpoint of ease of availability and quality stability, the hydrophobic organic solvent is preferably n-hexane, n-heptane, and cyclohexane. The hydrophobic organic solvent can also be used as a mixture solvent in which two or more of these hydrophobic organic solvents are mixed.

In the present invention, one or more dispersion aids selected from the group consisting of a surfactant, a polymer additive, and the like may be added, as necessary, to the hydrophobic organic solvent, provided that the object of the present invention is not impaired. The type of dispersion aid is selected as appropriate depending on a combination of the hydrophobic organic solvent and the monomer to be used. Examples of the dispersion aid that can be used encompass the following surfactants and polymer additives.

Specific examples of the surfactant encompass sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hardened castor oil, alkylallylformaldehyde-condensed polyoxyethylene ethers, polyoxyethylene polyoxypropylene block copolymers, polyoxyethylene polyoxypropyl alkyl ethers, polyethylene glycol fatty acid esters, alkyl glucosides, N-alkyl gluconamides, polyoxyethylene fatty acid amides, polyoxyethylene alkylamines, phosphates of polyoxyethylene alkyl ethers, phosphates of polyoxyethylene alkyl aryl ethers, and the like. Two or more of these surfactants may be used in combination. Further, a polymerizable surfactant having polymerizability can also be used. The polymerizable surfactant is, specifically, exemplified by a compound having the following structure:

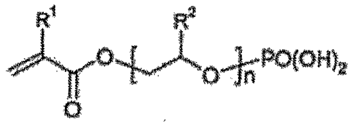

Note that, in the formula, $R^1$ and $R^2$ are hydrogen, methyl, or ethyl independently of each other, and n means an integer of 3 to 20. Among the above-described surfactants, fatty acid esters such as sucrose fatty acid esters, polyglycerin fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, and polyethylene glycol fatty acid esters are preferable. Among them, sucrose fatty acid esters having a hydrophile-lipophile balance (HLB) in the range of preferably 1 to 20, more preferably 1 to 10, and even more preferably 3 to 6 are preferable.

Specific examples of the polymer additive encompass maleic anhydride-modified polyethylene, maleic anhydride-modified polypropylene, maleic anhydride-modified ethylene-propylene copolymer, maleic anhydride-modified ethylene-propylene-diene terpolymer (EPDM), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, hydroxyethyl cellulose, and the like. Among them, from the viewpoint of dispersion stability of the aqueous monomer solution, preferable as the polymer additive are maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer. Two or more of these polymer additives may be used in combination. In addition, these polymer additives and the surfactant may be used in combination. Above all, it is preferable to use the polymer additive, and it is more preferable to use a maleic anhydride-modified ethylene-propylene copolymer. Further, in another preferred embodiment, the polymer additive is used solely without using the surfactant.

The amount of the dispersion aid used is set as appropriate in accordance with, for example, the polymerization form and the types of the aqueous monomer solution and of the hydrophobic organic solvent. Specifically, the concentration of the dispersion aid in the hydrophobic organic solvent is preferably 0.0001 mass% to 2 mass% and more preferably 0.0005 mass% to 1 mass%.

<3-3> Polymerization step

The polymerization step is a step of polymerizing droplets containing the monomer obtained in the dispersion step so that a hydrogel polymer (hereinafter also referred to simply as hydrogel) is obtained.

"Reaction apparatus"

As the reaction apparatus to be used in the polymerization step, the dispersion apparatus used in the dispersion step may be used as it is, or a different apparatus may be used. In the case of the batch-type reversed phase suspension polymerization, the apparatus used in the dispersion step can be used as it is as the reaction apparatus. This is suitable in terms of workability. In a case where the reaction apparatus is an apparatus different from the dispersion apparatus, the dispersion liquid of the monomer obtained in the dispersion step is supplied to the reaction apparatus.

Further, the shape of the reaction apparatus in which the polymerization reaction is carried out is not particularly limited, and a known reaction apparatus can be used. As described above, the stirring apparatus that can be suitably used in the dispersion step can also be suitably used in the polymerization reaction. In the case of the continuous-type production method, the shape of the reaction apparatus is preferably a shape that allows the monomer (aqueous solution) to undergo polymerization reaction while moving, as a droplet-like dispersed phase, in a hydrophobic organic solvent which is a continuous phase formed in this reaction apparatus. Examples of such a reaction apparatus encompass a reaction apparatus in which a tubular reaction tube is disposed in a vertical fashion, a horizontal fashion, or a spiral fashion. In this aspect, since the monomer (aqueous solution) is supplied into the hydrophobic organic solvent that moves in the reaction unit, the droplets composed of the aqueous monomer solution move together with the hydrophobic organic solvent without staying. This prevents contact between monomer reactants having different polymerization rates.

Further, the reaction apparatus may be provided with a temperature adjustment means so that the continuous phase inside the reaction apparatus can be heated or cooled from the outside, if necessary.

"Polymerization temperature"

A polymerization temperature, which is a reaction temperature in the polymerization step, is set as appropriate depending on the type and amount of the polymerization initiator to be used, and is preferably 20°C to 100°C, and more preferably 40°C to 90°C. The polymerization temperature of higher than 100°C is not preferable because such a polymerization temperature causes a rapid polymerization reaction. Note that the polymerization temperature means the temperature (hereinafter referred to as "Td") of the hydrophobic organic solvent that serves as a dispersion medium.

In the polymerization step, the monomer (aqueous solution) is dispersed in the hydrophobic organic solvent in the form of droplets. Thus, the temperature of the aqueous monomer solution rapidly increases due to heat transfer from the hydrophobic organic solvent. In a case where the polymerization initiator contained in the droplets is a pyrolytic polymerization initiator, the pyrolytic polymerization initiator is decomposed with the increase in temperature, so that radicals are generated. Then, the polymerization reaction is initiated by the generated radicals, and a hydrogel is formed as the polymerization reaction progresses.

In the case of the continuous-type production method, the formed hydrogel is moved inside the reaction apparatus by the moving continuous phase, and is discharged from the reaction apparatus together with the hydrophobic organic solvent that forms the continuous phase.

In a case where the aqueous monomer solution contains a pyrolytic polymerization initiator, the Td is preferably 70°C or higher, more preferably 75°C or higher, and even more preferably 80°C or higher, from the viewpoint of the polymerization rate. The upper limit of Td is not particularly limited and is selected as appropriate, from the viewpoint of safety, in a range which does not exceed the boiling point of the hydrophobic organic solvent that forms the continuous phase.

"Multi-stage reversed phase suspension polymerization"

In the production method in the present invention, multi-stage polymerization may be carried out from the viewpoint of obtaining an appropriate aggregated particle diameter. Specifically, the multi-stage polymerization can be carried out, for example, by, after the end of a first-stage polymerization step, further adding an aqueous monomer solution and carrying out a polymerization reaction.

"Inorganic fine particles"

In the production method in the present invention, inorganic fine particles may be added to the hydrogel polymer during the polymerization and/or after the end of the polymerization, from the viewpoint of obtaining an appropriate

aggregated particle diameter.

Examples of the inorganic fine particles that can be used in the present invention encompass silicon dioxide, aluminum oxide, titanium dioxide, calcium phosphate, calcium carbonate, magnesium phosphate, calcium sulfate, diatomaceous earth, bentonite, zeolite, other metal oxides, and the like. As the inorganic fine particles, silicon dioxide, aluminum oxide, and titanium dioxide in particular, are preferable.

Good results can be obtained when the amount of the inorganic fine particles added is such that the inorganic fine particles are used in a proportion of generally 0.001 parts by weight to 1 part by weight, preferably 0.001 parts by weight to 0.5 parts by weight, relative to 100 parts by mass of the hydrogel polymer. The amount of the inorganic fine particles added falling within this range is preferable since the effect produced by the addition of the inorganic fine particles is efficiently exhibited, and the influence on the water absorption performance is small.

<3-4> Separation step

The separation step is a step of separating the hydrogel polymer obtained in the polymerization step from the hydrophobic organic solvent. The type and structure of the apparatus to be used in the separation step are not particularly limited. For example, a known apparatus used for filtration, sedimentation, centrifugation, squeezing, and the like can be used. Further, separation of the hydrogel polymer from the hydrophobic organic solvent may be carried out by distillation performed through the heating of a mixture of the hydrogel polymer and the hydrophobic organic solvent under normal pressure or reduced pressure with use of the stirring apparatus having a stirring blade and used in the polymerization step. In the batch-type reversed phase suspension polymerization, distillation under normal pressure or reduced pressure is suitably performed.

<3-5> Gel sizing step

In the gel sizing step, the hydrogel polymer separated from the hydrophobic organic solvent in the separation step is sized with use of a gel sizing apparatus having an extrusion action unit and a porous plate. As a result, a sized hydrogel polymer (hereinafter, the hydrogel after gel sizing is referred to as sized gel) is obtained. The gel sizing step is an optional step. Having the gel sizing step makes it easy to control the bulk density of a water-absorbing resin.

The hydrogel polymer used in this gel sizing step is in the form of a single particle of a spherical gel or in the form of an aggregate of spherical gels. The lower limit of the average particle diameter of the hydrogel polymer is not particularly limited and is preferably 0.01 mm or more, more preferably 0.03 mm or more, even more preferably 0.05 mm or more, and still more preferably 0.1 mm or more. The upper limit of the average particle diameter of the hydrogel polymer is also not particularly limited and is preferably 20 mm or less and more preferably 10 mm or less. Further, in the case where the hydrogel polymer is in the form of a single particle, the particle diameter of the spherical gel is referred to as a primary particle diameter. In the case where the hydrogel polymer is in the form of an aggregate, the particle diameter of each spherical gel constituting the aggregate is referred to as a primary particle diameter. In the present invention, an average primary particle diameter is not particularly limited, and is preferably 5 pm to 2000 pm, more preferably 5 pm to 1000 pm, even more preferably 5 pm to 800 pm, still more preferably 8 pm to 500 pm, further more preferably 10 pm to 300 pm, and particularly preferably 10 pm to 200 pm, from the viewpoint of preventing the generation of fine powder when control is carried out to have the particle size of a final product. Note here that the average primary particle diameter is intended to mean a number average particle diameter.

Note that an apparatus having a cutter may be installed in front of the gel sizing apparatus having the extrusion action unit and the porous plate to crush large aggregates.

"Temperature of hydrogel"

The lower limit of the temperature of the hydrogel put into the gel sizing apparatus is not particularly limited, and is preferably 80°C or higher and more preferably 90°C or higher, from the viewpoint of sizing efficiency and prevention of damage to the hydrogel. The upper limit of the temperature of the hydrogel at the charge into the gel sizing apparatus is not particularly limited, and is generally 100°C or lower.

"Gel sizing apparatus"

In the present specification, "gel sizing" means an operation of extruding a wet powder mass into a columnar shape through a small hole of a porous plate to prepare grains having a substantially uniform shape and size from a wet powder-like raw material. That is, with use of the porous plate, hydrogels in the form of a coarse aggregate resulting from excessive aggregation in a solvent separation step which is a previous step are crushed, and hydrogels in the form of a single particle having a small particle diameter are appropriately aggregated. Therefore, this step makes it possible

to obtain a hydrogel (sized gel) having a granulation shape and having a relatively uniform particle diameter. The sized gel may contain a hydrogel in the form of a single particle.

As the "gel sizing apparatus having an extrusion action unit and a porous plate" used in the gel sizing step is not particularly limited, provided that the gel sizing apparatus is an apparatus (for example, an extruder) having an extrusion action unit and a porous plate (die or screen), the extrusion action unit ordinarily having an extrusion member for extruding and supplying the contents toward the porous plate, and being capable of preparing grains of a certain size by extruding a material from the porous plate. Further, a configuration may be employed in which these apparatuses are placed in series and used.

Further, the shape of the hole of this porous plate (die or screen) is not particularly limited, and it is possible to freely select a shape suitable for use, including a perfect-circular shape, an elliptical shape, a polygonal shape such as a hexagonal shape, a triangular shape, and the like. From the viewpoint of sizing strength, the shape of the hole is preferably a perfect-circular shape and an elliptical shape. The hole diameter of the hole is also not particularly limited. The hole diameter is preferably 1.5 mm or less, more preferably 1.0 mm or less, and even more preferably 0.8 mm or less. A hole diameter equal to or lower than such an upper limit prevents an increase in the size of a resulting sized gel more than necessary, and makes it possible to reduce the amount of fine powder generated when control is carried out to have the particle size of a final product. The hole diameter is preferably 0.3 mm to 1.5 mm and more preferably 0.3 mm to 0.8 mm. With the porous plate having a hole diameter of 0.3 mm or more, it is possible to efficiently carry out extrusion when an extrusion operation is carried out. Note that the hole diameter is defined as follows. First, in a case where the hole of the porous plate does not have a perfect-circular shape, a geometric mean value of a minor axis of the hole and a major axis thereof is adopted as the hole diameter. Further, in a case where the holes of the porous plate have different hole diameters, the hole diameters of all the holes are calculated, and an arithmetic mean value of the hole diameters is adopted as the hole diameter of the holes of the porous plate. Furthermore, in a case where the porous plate has varying hole diameters in a distance between an extrusion action unit side of the porous plate and a side opposite to the extrusion action unit side (the hole diameter varies in the thickness direction of the porous plate), a smallest hole diameter value among the hole diameters is adopted.

In this step, an additive may be further added. Examples of the additive that can be added in this step encompass a polymerization initiator, an oxidizing agent, a reducing agent, a chelating agent, a thickener, a surfactant, a crosslinking agent, an acid, a base, a foaming agent, organic or inorganic fine particles, a polyvalent metal salt, and the like. Among them, preferable as an additive that can control the degree of aggregation are, for example, a thickener such as starch, cellulose, a starch-cellulose derivative, and polyvinyl alcohol, a surfactant, fine powder of a water-absorbing resin, a crosslinking agent, a polyvalent metal salt, and the like.

<3-6> Drying step

The drying step is a step of drying a hydrogel.

In the present invention, as a drying method in the drying step, azeotropic dehydration in a hydrophobic dispersing solvent used in the conventional reversed phase suspension polymerization may be employed. As the drying method, as a more suitable method, for obtaining the water-absorbing resin in the present invention by reversed phase suspension polymerization, both stirring drying and static drying can preferably be employed instead of azeotropic dehydration. Among them, static drying is preferably employed in order to maintain the particle diameter of the gel controlled in the gel sizing step.

The dried polymer composed of particles obtained in this drying step can be directly used as a water-absorbing resin for each application. Further, in a case where a water-absorbing resin is produced by this production method, the dried polymer obtained in the drying step can be subjected to the surface-crosslinking step described later. In this case, the dried polymer to be subjected to the surface-crosslinking step described later is also referred to as "water-absorbing resin powder" for convenience.

"Additive"

An additive may be added to a hydrogel, provided that the effect of the present invention is not impaired. The addition may be carried out during heating by a heating means and/or during stirring (rotation) by a rotary container or may be carried out before the drying step (before heating by the heating means and/or before stirring (rotation) by the rotary container). Furthermore, the additive may be added in any step previous to the drying step. With the additive, excessive adhesion between hydrogels during drying can be reduced, and a water-absorbing resin having an excellent water absorption speed can be obtained.

<3-7> Hydrophilization treatment step

The hydrophilization treatment step is a step of subjecting, to hydrophilization treatment, a water-absorbing resin obtained through the separation step and the drying step (and the subsequent optional step). Further, the hydrophilization treatment step may be carried out after the surface-crosslinking step described later or before the surface-crosslinking step. The hydrophilization treatment step is a step of hydrophilizing the surface of a water-absorbing resin and means that the wettability of the surface is improved after the hydrophilization treatment step. A specific method for hydrophilization treatment is as follows. In a case where a water-absorbing resin has been produced by reversed phase suspension polymerization, a surfactant used as a dispersant during polymerization is the cause of hydrophobicity. Thus, it is preferable to clean the surfactant, and it is preferable to clean the surfactant with use of an organic solvent. The hydrophilization treatment makes it possible to enhance the effect of surface-crosslinking treatment (the effect of increasing absorption capacity of a water-absorbing resin under pressure and reducing the amount of return). In addition, since the surface of the water-absorbing resin becomes highly hydrophilic, the water absorption speed also increases.

"Organic solvent"

The organic solvent is not particularly limited, provided that the organic solvent is capable of cleaning the dispersant. In order to enhance a cleaning effect, it is preferable to use an organic solvent that does not cause a water-absorbing resin to swell. A preferable swelling capacity of a water-absorbing resin during the treatment is less than twice. As the organic solvent, not only a hydrophilic organic solvent but also a hydrophobic organic solvent can be used. Specific examples of the hydrophilic organic solvent encompass: lower alcohols such as methyl alcohol, ethyl alcohol, n-propyl alcohol, isopropyl alcohol, n-butyl alcohol, isobutyl alcohol, and t-butyl alcohol; ketones such as acetone; ethers such as dioxane and tetrahydrofuran; amides such as N,N-dimethylformamide; sulfoxides such as dimethyl sulfoxide; and the like. Examples of the hydrophobic organic solvent encompass: aliphatic hydrocarbons like n-pentane, n-heptane, n-hexane, n-octane, and the like; alicyclic hydrocarbons like cyclohexane, methylcyclohexane, cyclooctane, decalin, and the like; halogenated hydrocarbons like chlorobenzene, bromobenzene, carbon tetrachloride, 1,2-dichloroethane, and the like; aromatic hydrocarbons like benzene, toluene, xylene, and the like; and the like. Among these organic solvents, methyl alcohol, ethyl alcohol, isopropyl alcohol, n-hexane, and cyclohexane are preferably used.

In a case where a water-absorbing resin is subjected to hydrophilization treatment with an organic solvent, bringing the organic solvent in a heated state into contact with the water-absorbing resin is preferable because the absorption capacity under pressure and/or the amount of return may be further improved. A heating temperature is preferably equal to or lower than a boiling point of the organic solvent, varies depending on the type of the organic solvent used, and is generally about 40°C to 120°C. The hydrophilization treatment is preferably carried out in a state in which a water-absorbing resin is dry. In a case where the hydrophilization treatment is carried out in a state in which a water-absorbing resin is moistened with an organic solvent and/or water, caution must be exercised. This is because a decrease in water absorption speed and/or a sharp decrease in water absorption capacity may be caused in a case where such a water-absorbing resin is subjected to crosslinking treatment at and near the surface thereof. Therefore, it is preferable to carry out the hydrophilization treatment in a state in which a solvent for polymerization and/or moisture are removed through filtering and drying of the water-absorbing resin obtained by reversed phase suspension polymerization.

<3-8> Surface-crosslinking step

The water-absorbing resin (for example, water-absorbing resin powder) obtained through the drying step (and the subsequent optional step) is preferably surface-crosslinked with a surface-crosslinking agent. This surface crosslinking is a process of providing a portion having a high crosslinking density on a surface layer (a portion within several tens of micrometers from the surface of water-absorbing resin (for example, water-absorbing resin powder)) of the water-absorbing resin (for example, water-absorbing resin powder). Various water absorption properties can be improved by carrying out surface-crosslinking treatment. Here, adjusting the crosslinking density as appropriate makes it possible to obtain excellent absorption capacity under pressure in particular.

Note that, in the present invention, known surface crosslinking techniques are employed as appropriate, including surface crosslinking in a state dispersed in a hydrophobic organic solvent, which is carried out in the conventional reversed phase suspension polymerization, and/or surface crosslinking to powder in a dry state, which is generally carried out in aqueous solution polymerization. Among them, in the present invention, surface crosslinking to a water-absorbing resin that has undergone the gel separation step and the drying step is preferable from the viewpoint of improvement in absorption capacity under pressure.

Note that the surface-crosslinking agent used in this step is also presented as a "postcrosslinking agent" in the known technique in order to be distinguished from the internal crosslinking agent used in the aqueous monomer solution preparation step.

In the present invention, the surface-crosslinking step may be carried out after the above-described drying step or during the drying step. In a known surface-crosslinking step, a surface-crosslinking agent is generally mixed with a hydrogel crosslinked polymer or a crosslinked polymer which is a dried product thereof, and the mixture is heated to carry out a crosslinking reaction. In contrast, in the present invention, these steps may be separately provided after the above-described drying step, or a surface-crosslinking agent may be added in the drying step to simultaneously carry out surface-crosslinking reaction and drying. Further, in a case where a water-absorbing resin is produced by a batch-type reversed phase suspension polymerization method, the solvent and the hydrogel polymer can be separated by carrying out distillation in the separation step after the polymerization reaction. By adding a surface-crosslinking agent even in the middle of the separation step, a surface-crosslinked water-absorbing resin (for example, water-absorbing resin particles) can be obtained.

<3-9> Other steps

A method for producing a water-absorbing resin in accordance with the present invention can include, in addition to the above-described steps, optionally a cooling step, a pulverizing step, a hydration (re-moistening) step, an other additive addition step, a classification step, a sizing step, and a fine powder reuse step. Further, the method may further include a conveying step, a storing step, a packing step, a reserving step, and the like.

(Cooling step)

In the cooling step carried out optionally, the particulate dried polymer obtained in the drying step is cooled with use of a known cooling means, so that a particulate dried polymer cooled to a desired temperature can be obtained.

(Pulverizing step)

The water-absorbing resin may undergo the pulverizing step of pulverizing the particulate dried polymer obtained in the drying step (and the subsequent optional cooling step). By undergoing the pulverization step, water-absorbing resin powder having a controlled particle diameter or a controlled particle size distribution is obtained.
In the pulverizing step, for example, a high-speed rotation pulverizer such as a roll mill, a hammer mill, a screw mill, or a pin mill; a vibration mill; a knuckle-type pulverizer; a cylindrical mixer; or the like is selected as appropriate and used as a pulverizing means.

(Re-moistening step)

This step carried out optionally is a step of adding, to the water-absorbing resin (for example, water-absorbing resin particles) obtained in the surface-crosslinking step, at least one additive selected from the group consisting of a polyvalent metal salt, a cationic polymer, a chelating agent, an inorganic reducing agent, $\alpha$-hydroxycarboxylic acid compound, a deodorizing agent, and an antibacterial agent.
The above-described additive in the form of an aqueous solution or a dispersion (slurry) is preferably added to a water-absorbing resin (for example, water-absorbing resin particles). Note that the additive may be added and mixed, together with the above-mentioned surface-crosslinking agent solution.
Specifically, a method described in "(2-7) Re-moistening step" of International Patent Publication No. WO 2015/053372 is also employed in the present invention.

(Other additive addition step)

In the present invention, an additive other than the above-described additives can be added in order to give various functions to the water-absorbing resin. Specifically, examples of such an additive encompass a surfactant, a compound having a phosphorus atom, an oxidizer, an organic reducing agent, water-insoluble inorganic fine particles, organic powder such as metallic soap, a deodorizing agent, an antibacterial agent, pulp, thermoplastic fibers, and the like. Note that, as the water-insoluble inorganic fine particles, a compound disclosed in "<5> Water-insoluble inorganic fine particles" of International Publication No. WO 2011/040530 is employed in the present invention. Among these additives, water-insoluble inorganic fine particles are preferable. Hydrophilic fine particles in particular, for example, silica (silicon dioxide), are preferably added because a resulting water-absorbing resin (for example, water-absorbing resin particles) has an improved compatibility with a liquid, and the water-absorbing resin, when used in an absorbent article, can absorb a water-based liquid in a short time.

(Sizing step)

The "sizing step" means a step of adjusting the particle diameter by disintegrating a water-absorbing resin having been loosely aggregated through the surface-crosslinking step. Note that this sizing step encompasses a fine powder removal step and a classification step, which are steps subsequent to the surface-crosslinking step. The sizing step is preferably carried out from the viewpoint of adjusting the particle diameter of a water-absorbing resin and obtaining stable water absorption physical properties.

(Fine powder reuse step)

The "fine powder reuse step" means a step of supplying, to any of the steps, the fine powder generated by, for example, sieve classification in each of the above steps as it is or after having granulated the fine powder. The fine powder reuse step is preferably carried out from the viewpoint of reducing a production loss of a water-absorbing resin.

<4> Absorbent article and method for producing the same

An absorbent article in accordance with an embodiment of the present invention includes: a liquid-permeable top sheet; an absorbent body containing a water-absorbing resin in the present invention; and a liquid-impermeable back sheet in this order, and a basis weight of the water-absorbing resin is 300 $g/m^2$ to 1000 $g/m^2$.

A method for producing an absorbent article in accordance with an embodiment of the present invention includes a step of disposing, between a liquid-permeable top sheet and a liquid-impermeable back sheet, an absorbent body containing a water-absorbing resin in the present invention, and a basis weight of the water-absorbing resin is 300 $g/m^2$ to 1000 $g/m^2$.

A method for producing an absorbent article in accordance with an embodiment of the present invention includes: a step of selecting a water-absorbing resin in the present invention; and a step of disposing, between a liquid-permeable top sheet and a liquid-impermeable back sheet, an absorbent body containing the selected water-absorbing resin, and a basis weight of the water-absorbing resin is 300 $g/m^2$ to 1000 $g/m^2$.

An absorbent article in accordance with an embodiment of the present invention is not particularly limited, and preferably includes disposable diapers (for infants and for adults), sanitary napkins, incontinence pads, and the like. Particularly, the absorbent article in accordance with an embodiment of the present invention can be used as thin high-concentration disposable diapers. Other examples of the absorbent article in accordance with an embodiment of the present invention include: for example, raising sheets; dew condensation prevention sheets; drip absorbers; freshness-keeping materials; disposable body warmers; cooling bandanas; ice packs; absorption sandbags; fomentation materials; waterproof materials for electric and electronic material communication cables; gasket packings; pet sheets; dressing materials for wound protection; building materials for dew condensation prevention; moisture-in-oil removers; and the like.

In an embodiment of the present invention, the basis weight of the water-absorbing resin means the weight of the water-absorbing resin per unit area of the absorbent article. Further, the basis weight of the water-absorbing resin is also the weight of the water-absorbing resin per unit area of the absorbent body that constitutes the absorbent article. The basis weight of the water-absorbing resin equal to or greater than a specific value suitably improves absorption performance of the absorbent article. From the viewpoint of suitably reducing or preventing leakage of an added water-based liquid from sides of an absorbent article due to the occurrence of the liquid running along the crease, the basis weight of the water-absorbing resin is 300 $g/m^2$ or more, preferably 350 $g/m^2$ or more, and more preferably 380 $g/m^2$ or more. Further, the basis weight of the water-absorbing resin equal to or less than a specific value prevents the absorbent article from becoming excessively thick and increasing in size. From the viewpoint of preventing the absorbent article from becoming excessively thick and increasing in size, the basis weight of the water-absorbing resin is 1000 $g/m^2$ or less, preferably 700 $g/m^2$ or less, and more preferably 500 $g/m^2$ or less.

In an embodiment of the present invention, the top sheet is not particularly limited. A known sheet that can be used as a liquid-permeable sheet constituting an absorbent article can be used as the top sheet. The liquid-permeable top sheet is ordinarily a nonwoven fabric, and may be, for example, an air-through nonwoven fabric, a point bond nonwoven fabric, a spunbond nonwoven fabric, a spunlace nonwoven fabric, and the like. Further, a composite nonwoven fabric of these nonwoven fabrics can also be used as the liquid-permeable top sheet. The composite nonwoven fabric include, for example, a spunbond-meltblown-meltblown-spunbond nonwoven fabric (SMMS nonwoven fabric). These are preferably subjected to hydrophilization treatment with a surfactant.

A substance constituting the top sheet is not particularly limited, and may include, for example, synthetic fibers such as polypropylene, polyethylene, and polyester.

In an embodiment of the present invention, the back sheet is not particularly limited. A known sheet that can be used as a liquid-impermeable sheet constituting an absorbent article can be used as the back sheet. As the liquid-impermeable back sheet, for example, thin plastic films such as a polyethylene film can be used. Among the plastic

films, an air-permeable film is preferable in order to provide comfort to a user of the absorbent article.

An absorbent body in accordance with an embodiment of the present invention contains the water-absorbing resin. Further, the absorbent body may include, in addition to the water-absorbing resin, a hydrophilic fiber material and/or an additive. As the additive, an additive that can be generally included in an absorbent body of an absorbent article can be used. The additive is not particularly limited. Specific examples of the additive include, for example, inorganic powder (for example, amorphous silica), a deodorizing agent, a pigment, a dye, an anti-bacterial agent, a perfume, an adhesive agent, and the like. In a case where the water-absorbing resin contains inorganic particles, the absorbent body may contain inorganic powder separately from the inorganic particles in the water-absorbing resin. Examples of the inorganic powder include silicon dioxide, zeolite, kaolin, clay, and the like.

A method for preparing an absorbent body in accordance with an embodiment of the present invention is not particularly limited. As such a method, a generally known method as a method for preparing an absorbent body of an absorbent article can be employed. Specific examples of the method for preparing an absorbent body include, for example, a method of obtaining a mixture by mixing a water-absorbing resin in the present invention and a hydrophilic fiber material and shaping the mixture, a method of forming a layer of hydrophilic fibers and then spraying the water-absorbing resin in the present invention on the layer of hydrophilic fibers, a method of spraying a water-absorbing resin in the present invention in the form of a layer to form a layer of absorbent resin and then placing the layer of hydrophilic fibers on the layer of water-absorbing resin, and the like method. The absorbent body prepared in this manner may be a uniform mixture of the water-absorbing resin in the present invention and hydrophilic fibers. Alternatively, the water-absorbing resin in the present invention and hydrophilic fibers may be formed in the form of respectively different layers in contact with each other. In a case where the water-absorbing resin in the present invention and hydrophilic fibers may be formed in the form of respectively different layers, the water-absorbing resin in the present invention and the hydrophilic fibers may each be formed in the form of one layer. Alternatively, at least one of the water-absorbing resin in the present invention and the hydrophilic fibers may be formed in the form of a plurality of layers so that the layer of the water-absorbing layer of the present invention and the layer of the hydrophilic fibers are alternately laminated. Even in a case where the water-absorbing resin in the present invention and the hydrophilic fibers are in the form of respectively different layers, the water-absorbing resin in the present invention may be mixed in the layer of the hydrophilic fibers. The absorbent body of the absorbent article in accordance with an embodiment of the present invention preferably has a layer of hydrophilic fibers on a back sheet side and a layer of a water-absorbing resin on a top sheet side in terms of the thickness of the absorbent article and the diffusibility of the absorbed water-based liquid because the water-absorbing resin is mixed into the layer of hydrophilic fibers when the absorbent article is used. The absorbent body of the absorbent article in accordance with an embodiment of the present invention preferably has another layer of hydrophilic fibers on the top sheet side from the viewpoint of preventing liquid running. That is, the layer of the water-absorbing resin is sandwiched between the layers of hydrophilic fibers. The obtained absorbent article is packaged after production, and further packaged in a folded state (two-fold, three-fold, etc.) and distributed and sold. Therefore, the absorbent article in accordance with an embodiment of the present invention can be an absorbent article packaged in a folded state.

The hydrophilic fiber material is not particularly limited, and examples of the hydrophilic fiber material encompass pulp fibers, cotton linter, crosslinked cellulose fibers, rayon, cotton, wool, acetate, vinylon, and the like. Further, in an embodiment of the present invention, the above-described hydrophilic fiber material is preferably a hydrophilic fiber material obtained by air-laying the hydrophilic fiber materials listed above.

As the hydrophilic fiber material, an absorbent material such as pulp fibers can also be used. In such a case, the content (core concentration) of the water-absorbing resin in the absorbent body is preferably 30 mass% or more and less than 100 mass%, more preferably 40 mass% or more and less than 100 mass%, even more preferably 50 mass% or more and less than 100 mass%, further more preferably 60 mass% or more and less than 100 mass%, particularly preferably 70 mass% or more and less than 100 mass%, and most preferably 75 mass% or more and 95 mass% or less. However, in the present invention, the hydrophilic fiber material used in the absorbent body does not include a fabric-like material (woven fabric or nonwoven fabric).

In a case where the absorbent body having the core concentration in the above range is used as an upper layer part of an absorbent article, the absorbent article can maintain cleanness, i.e., a state of being white. Further, such an absorbent article is excellent in diffusion property with respect to a body fluid or the like such as urine and blood and thus achieves efficient liquid distribution. Therefore, improvement in absorption amount in the absorbent article in accordance with an embodiment of the present invention can be expected.

More specifically, the absorbent body in accordance with an embodiment of the present invention may be an absorbent body which contains, in addition to a water-absorbing resin in accordance with an embodiment of the present invention, a hydrophilic fiber material and in which the mass of the water-absorbing resin accounts for 50 mass% or more and less than 100 mass% of a total mass of the water-absorbing resin and the hydrophilic fiber material.

In a case where the mass of the water-absorbing resin accounts for 50 mass% or more of the total mass of the water-absorbing resin and the hydrophilic fiber material, more specifically, the mass of the water-absorbing resin may account for 60 mass% or more, 70 mass% or more, 80 mass% or more, 90 mass% or more, less than 100 mass%, or

100 mass% of the total mass of the water-absorbing resin and the hydrophilic fiber material.

In a method for producing an absorbent article in accordance with an embodiment of the present invention, a method of selecting the water-absorbing resin in the present invention is not particularly limited and can include, for example, a method in which a water-absorbing resin is partially split, a portion of the water-absorbing resin obtained by the split is subjected to measurement in bulk density and measurements in vertical and horizontal liquid diffusion lengths, and, after it has been confirmed that the water-absorbing resin is the water-absorbing resin in the present invention, a remaining water-absorbing resin is used as a raw material for the absorbent article.

<Summary>

The present invention can include the following inventions <1> to <8>.

<1> An absorbent article including a liquid-permeable top sheet, an absorbent body containing a water-absorbing resin, and a liquid-impermeable back sheet in this order, wherein

a basis weight of the water-absorbing resin is 300 $g/m^2$ to 1000 $g/m^2$, and
the water-absorbing resin is a particulate poly(meth)acrylic acid (salt)-based water-absorbing resin and has the following physical property values (1) to (3):

(1) a vertical liquid diffusion length (D) is 15 mm or more;
(2) a ratio (L/D) of a horizontal liquid diffusion length (L) to the vertical liquid diffusion length is 0.5 to 1.5; and
(3) a bulk density defined in EDANA ERT 460.2-02 is 0.68 g/mL or more,

where the vertical liquid diffusion length (D) and the horizontal liquid diffusion length (L) are determined by filling a transparent container made of an acrylic resin (having internal dimensions of 40 mm in a vertical direction (height) by 72 mm in a horizontal direction (width) by 3 mm in depth) with the water-absorbing resin up to a position at a height of 30 mm, injecting, from a surface of the water-absorbing resin, 1 mL of a 0.9 mass% aqueous sodium chloride solution which is colored and has a temperature of 20°C, and measuring a diffusion length in the vertical direction and a diffusion length in the horizontal direction 1 minute after the injection.

<2> The absorbent article described in <1>, wherein the water-absorbing resin is an aggregate-like particle of spherical particles.
<3> The absorbent article described in <1> or <2>, wherein the water-absorbing resin includes water-insoluble inorganic fine particles.
<4> The absorbent article described in any one of <1> to <3>, wherein the water-absorbing resin has a mass average particle diameter (D50) of 200 pm to 700 pm.
<5> The absorbent article described in any one of <1> to <4>, wherein the water-absorbing resin has an AAP of 18 g/g or more.
<6> The absorbent article described in any one of <1> to <5>, wherein the water-absorbing resin has a bulk density defined in EDANA ERT 460.2-02 of 0.70 g/mL or more.
<7> The absorbent article described in any one of <1> to <6>, wherein the absorbent body further contains a hydrophilic fiber material, and a mass of the water-absorbing resin accounts for 50 mass% or more and less than 100 mass% of a total mass of the water-absorbing resin and the hydrophilic fiber material.
<8> The absorbent article described in any one of <1> to <7>, wherein the absorbent article is packaged in a folded state. Examples

The following description will discuss the present invention more concretely with reference to Examples and Comparative Example shown below. Note, however, that the present invention is not limited to these Examples and Comparative Example, and that any Example derived from a proper combination of technical means disclosed in respective different Examples is also encompassed in the scope of the present invention. Note that "part(s)" and "%" may be used in Examples, and "part(s)" and "%" indicate "part(s) by mass" and "mass%", respectively, unless otherwise noted. In addition, the operations are carried out at room temperature (25°C) unless otherwise specified. Note that electric apparatuses (including apparatuses used to measure physical properties of water-absorbing resins) used in Production Examples, Examples, and Comparative Examples each used a 200-V or 100-V electric power supply with a frequency of 60 Hz, unless otherwise specified.

<Evaluation method>

[Number average particle diameter]

A scanning electron microscope photograph (SEM) of a water-absorbing resin or water-absorbing resin powder was taken. From the photograph, 50 primary particles on the front of the aggregate-like particle were randomly selected. The major axis and minor axis of each particle were measured, and an average value of the measured values was assumed to be a primary particle diameter. An average value of the primary particle diameters of the particles was calculated, and the average value was assumed to be a number average particle diameter of the water-absorbing resin.

[Moisture content]

The moisture content was measured in conformity with an EDANA method (ERT 430.2-02). Note that, at the time of the measurement, the mass of a sample (water-absorbing resin) was changed to 1.0 g, the drying temperature was changed to 180°C, and the drying time was changed to 3 hours. Specifically, 1.0 g of water-absorbing resin or water-absorbing resin powder was put into an aluminum cup having a 50-mm-diameter bottom surface, and then a total mass W1 (g) of the sample (water-absorbing resin or water-absorbing resin powder) and the aluminum cup was weighed accurately. Next, the sample was allowed to stand still in an oven set to an atmospheric temperature of 180°C in a state in which the sample was put into the aluminum cup. After a lapse of 3 hours, the sample was taken out from the oven together with the aluminum cup, and a total mass W2 (g) of the sample and the aluminum cup after drying was weighed accurately. When the mass of the sample (water-absorbing resin) used in this measurement was assumed to be M (1.0 g), the moisture content $\alpha$ (mass%) of the sample was determined in accordance with the following (Formula 1):

$$\text{Moisture content } \alpha \text{ (mass\%)} = \{(W1 - W2)/M\} \times 100$$

Formula (1).

[Mass average particle diameter (D50)]

The mass average particle diameter (D50) of a water-absorbing resin was measured in accordance with a method described in "(3) Mass-Average Particle Diameter (D50) and Logarithmic Standard Deviation ($\sigma\zeta$) of Particle Diameter Distribution" described in Columns 27 and 28 of US Patent No. 7,638,570.

[CRC]

The centrifuge retention capacity (CRC) of a water-absorbing resin was measured in conformity with an EDANA method (ERT 441.2-02). Specifically, a water absorption capacity (unit: g/g) was determined after 0.2 g of water-absorbing resin contained in a nonwoven fabric bag was immersed in a large excess of a 0.9 mass% aqueous sodium chloride solution for 30 minutes so as to be allowed to freely swell, and then the water-absorbing resin was drained for 3 minutes with use of a centrifuge (250 G).

[AAP]

The AAP (absorption capacity under pressure) of a water-absorbing resin was measured in conformity with an EDANA method (ERT 442.2-02). Note that the load condition was changed to 4.83 kPa (0.7 psi).

[Ext]

The Ext (water-soluble component) of a water-absorbing resin was measured in conformity with an EDANA method (ERT 470.2-02).

[Vertical liquid diffusion length and horizontal liquid diffusion length]

The liquid diffusion lengths of a water-absorbing resin were measured by the procedure presented below.

Fig. 1 is a perspective view illustrating a container used for measuring the liquid diffusion lengths. A water-absorbing resin was poured little by little into a transparent container 2 made of an acrylic resin (having internal dimensions of 40 mm in a vertical direction (height) by 72 mm in a horizontal direction (width) by 3 mm in depth). While the water-absorbing resin was leveled horizontally by a spatula, the transparent container 2 was filled with the water-absorbing resin up to a position at a height of 30 mm.

With use of a syringe (syringe needle gauge: 21 G), 1 mL of a 0.9 mass% aqueous sodium chloride solution colored in blue and having a temperature of 20°C was gently injected over 5 seconds from a central portion (arrow A in Fig. 1) of a top surface (3 mm × 72 mm surface) of the filled water-absorbing resin. After 1 minute, the vertical and horizontal diffusion lengths [mm] of the 0.9 mass% aqueous sodium chloride solution colored in blue and having a temperature of 20°C were measured.

Fig. 2 is a schematic view for describing the measurement of the liquid diffusion lengths, and is a view of the container 2 which was filled with a water-absorbing resin 1 as viewed from the lateral side. In a colored region 3 colored by the aqueous sodium chloride solution (in a case where the water-absorbing resin 1 was swollen upward by the liquid injection, the colored region 3 also includes the swollen portion), the longest diffusion distance in the vertical direction and the longest diffusion distance in the horizontal direction were defined as the vertical liquid diffusion length D and the horizontal liquid diffusion length L, respectively.

[Bulk density]

The bulk density of a water-absorbing resin in the present invention was measured in conformity with an EDANA method (T460.2-02).

[Surface tension]

The surface tension in the present invention was measured by a method described in WO 2015/129917. Specifically, 50 ml of a 0.90 weight% aqueous sodium chloride solution adjusted to 20°C was put into a 100 ml beaker which had been sufficiently washed. Then, first, the surface tension of the 0.90 weight% aqueous sodium chloride solution adjusted to 20°C in the beaker was measured with use of a surface tension meter (K11 automatic surface tension meter manufactured by KRUSS). In this measurement, a value of the surface tension needs to fall within a range of 71 mN/m to 75 mN/m. Next, a 25 mm-long cylindrical stirrer which had been sufficiently washed and 0.500 g of a particulate water-absorbing agent were put into the beaker containing the 0.90 weight% aqueous sodium chloride solution adjusted to 20°C and subjected to the surface tension measurement. Then, a resulting solution was stirred at 500 rpm for 4 minutes. After 4 minutes, the stirring was stopped. After sedimentation of the particulate water-absorbing agent which had absorbed water, the surface tension of a supernatant liquid was measured again by performing a similar procedure, i.e. with use of the surface tension meter. Note that, in the present Example, a plate method using a platinum plate was employed, and the plate was sufficiently washed with deionized water and also cleaned with heat by the use of a gas burner before being used in each of the above measurements.

[Amount of liquid return from absorbent sheet]

(1) Preparation of absorbent sheet

Fig. 3 is a view illustrating an absorbent sheet 4 as viewed from directly above, and Fig. 4 is a cross-sectional view illustrating the absorbent sheet 4. On the adhesive surface of an adhesive tape 6 (vinyl tape No. 21S available from NITTO DENKO) cut to 10 cm × 10 cm, 1.0 g of the water-absorbing resin 1 was spread over an area 1 cm inward of an edge on each of the four sides of the adhesive tape 6, a 10 cm × 10 cm nonwoven fabric 5 (air-laid nonwoven fabric having a basis weight of 46.6 g/m$^2$) was placed. The nonwoven fabric 5 and the adhesive surfaces of the four sides of the adhesive tape 6 were bonded together to prepare the absorbent sheet 4 for evaluation.

(2) Measurement of amount of return

The absorbent sheet 4 for evaluation was placed on a flat table, and 10 mL of a 0.9 mass% aqueous sodium chloride solution having a temperature of 20°C was added to the absorbent sheet 4 for evaluation over 20 seconds from a position 1 cm vertically above the central portion of the absorbent sheet 4 for evaluation. 30 seconds after the start of the addition of 10 mL of the aqueous sodium chloride solution, 10 sheets of filter paper (ADVANTEC 2 Φ 55 mm) and a weight of 1.0 kg were placed in the central portion of the absorbent sheet for evaluation. After 10 seconds, the sheets of filter paper and the weight were removed, and an increase in weight of the sheets of filter paper (weight of liquid absorbed by the filter paper) was regarded as the amount of liquid return.

"Basis weight of water-absorbing resin"

With use of the area and weight of an absorbent body used in each of Examples and Comparative Examples described later and the content (core concentration) of a water-absorbing resin in the absorbent body, the basis weight of the water-absorbing resin in the absorbent article was calculated on the basis of the following formula (3):

$$\{\text{Weight (g) of absorbent body} \times \text{content (weight\%) of water-absorbing resin} \times 10^{-2}\}/\text{area (m}^2\text{) of absorbent body} = \text{basis weight (g/m}^2\text{) of water-absorbing resin} \quad (3)$$

"Liquid diffusion lengths of absorbent article"

As illustrated in Figs. 5 and 6, a central portion of an absorbent article obtained in each of Examples and Comparative Examples in the longitudinal direction (lengthwise direction) of the absorbent article was fixed in a state in which the absorbent article was bent into a 45° angle in an extended upward direction to obtain an absorbent article with a crease. Note that the absorbent article with the crease was fixed on a stand such that a portion of the absorbent article on the lower side thereof from the crease as a boundary is a bottom portion of the absorbent article. Subsequently, a 0.9 mass% aqueous sodium chloride solution (hereinafter referred to as physiological saline) colored in blue was charged by flowing the 0.9 mass% aqueous sodium chloride solution for 1 minute at a speed of 10.2 g/min from the height of 5 mm to the central portion of the absorbent article with the crease, that is, to the center of the crease via a liquid feed tube (inner diameter of 1 mm). For 30 seconds after the end of the charging of the physiological saline, the absorbent article into which the physiological saline had been charged was allowed to stand still. The absorbent article having been allowed to stand still absorbed the physiological saline colored in blue, and a portion where the physiological saline was diffused was colored in blue.

The respective lengths of the portion colored in blue in the absorbent article having been allowed to stand still in the longitudinal direction and in the lateral direction were measured and regarded respectively as the liquid diffusion length in the longitudinal direction and the liquid diffusion length in the lateral direction. Here, the longitudinal direction is a direction perpendicular to the crease in the absorbent article with the crease. Further, the lateral direction is a direction parallel to the crease in the absorbent article with the crease.

In addition, in the portion colored in blue in the absorbent article having been allowed to stand still, the length in the upward direction from the portion into which the physiological saline had been charged, that is, the center of the crease, and the length in the downward direction from the center of the crease were measured and regarded respectively as the liquid diffusion length in the upward direction from a liquid charging portion and the liquid diffusion length in the downward direction from the liquid charging portion. Here, the downward direction is a direction that extends to the side of a portion, from the center of the crease in the longitudinal direction, downward of the crease of the absorbent article as a boundary, that is, a direction that extends to the side of the bottom portion. Conversely, the upward direction is a direction that extends to the side of a portion, from the center of the crease in the longitudinal direction, upward of the crease of the absorbent article as a boundary, that is, a direction that extends to the side opposite from the bottom portion. The length of the portion colored in blue in the absorbent article having been allowed to stand still in the longitudinal direction is a sum of the liquid diffusion length in the upward direction from the liquid charging portion and the liquid diffusion length in the downward direction from the liquid charging portion.

[Reference Example 1]

800 g of cyclohexane was placed in a 2000-ml four-necked separable flask equipped with a stirrer, a reflux condenser, a thermometer, a nitrogen gas introduction tube, and a dropping funnel, 0.6 g of sucrose fatty acid ester (DK Ester (registered trademark) F-50, available from DKS Co., Ltd., HLB=6) as a dispersing agent was added and dissolved, and nitrogen gas was blown into a resulting solution to expel dissolved oxygen.

Separately from this, prepared in a flask was an aqueous monomer solution (1) composed of 141 g of sodium acrylate, 36 g of acrylic acid, 0.022 g of N,N'-methylenebisacrylamide, and 327.65 g of ion-exchanged water, and nitrogen gas was blown into the aqueous monomer solution (1) to expel dissolved oxygen dissolved in the aqueous monomer solution (1). Next, after 0.95 g of a 15% aqueous solution of sodium persulfate had been added to the aqueous monomer solution in this flask, the whole amount of a resulting solution was added to the separable flask. A resulting mixture solution was stirred at 415 rpm for dispersion. After that, the temperature of a bath was increased to 60°C to initiate a polymerization reaction. After this bath temperature of 60°C was maintained for 2 hours, the polymerization was stopped, filtration was carried out by suction filtration, and a residue was air-dried overnight to obtain a hydrogel polymer (1). The average primary particle diameter of the hydrogel polymer (1) was 100 μm.

Next, the hydrogel polymer (1) (gel temperature: 90°C) was charged into a gel sizing apparatus having a screw

and a porous plate having a hole diameter of 0.8 mm, and the hydrogel polymer (1) was discharged from the gel sizing apparatus to obtain a sized gel (1).

The sized gel (1) thus obtained was dried with use of a hot-air dryer to obtain a dried polymer (1). The drying was carried out by spreading, onto a stainless steel metal gauze having a mesh size of 850 pm, the sized gel (1) and allowing hot air of 105°C to pass through for 40 minutes.

The obtained dried polymer (1) was added to methanol (in an amount 10 times the weight of the dried polymer (1)) heated to 60°C and was stirred for 1 hour, followed by filtration and drying to carry out hydrophilization treatment.

Subsequently, the dried polymer (1) subjected to hydrophilization treatment was supplied to a roll mill (pulverizer) and pulverized to adjust the particle size, and water-absorbing resin powder (1) was obtained. The average particle diameter of the water-absorbing resin powder (1) was 70 pm.

To 100 parts by mass of the water-absorbing resin powder (1), a surface-crosslinking agent solution composed of 0.1 parts by mass of ethylene glycol diglycidyl ether, 1.0 part by mass of isopropyl alcohol, and 3.0 parts by mass of ion-exchanged water was sprayed with a spray, followed by uniform mixing with use of a high speed continuous mixer.

A resulting mixture was introduced into a heat treatment machine adjusted to an atmospheric temperature of 100°C and heat-treated for 40 minutes. Then, the powder temperature was forcibly cooled to 60°C to obtain surface-crosslinked water-absorbing resin powder (1).

To 100 parts by mass of the surface-crosslinked water-absorbing resin powder (1), 5.0 parts by mass of water was mixed uniformly. A resulting mixture was sized by passage through a JIS standard sieve having a mesh size of 300 pm to obtain water-absorbing resin particles (1).

To 100 parts by mass of the obtained water-absorbing resin particles (1), 0.3 parts by mass of silicon dioxide (trade name: Aerosil (registered trademark) 200; available from Nippon Aerosil Co., Ltd.) in the form of fine particles was added and mixed to obtain a water-absorbing resin (1). Tables 1 to 3 show physical properties of the obtained water-absorbing resin (1).

[Reference Example 2]

800 g of n-heptane was placed in a 2000-ml four-necked separable flask equipped with a stirrer, a reflux condenser, a thermometer, a nitrogen gas introduction tube, and a dropping funnel, 0.88 g of maleic anhydride-modified ethylene-propylene copolymer as a dispersing agent (trade name: Hi-WAX (registered trademark) HW2203A; available from Mitsui Chemicals Co., Ltd.) was added and dissolved, and nitrogen gas was blown into a resulting solution to expel dissolved oxygen.

Separately from this, prepared in a flask was an aqueous monomer solution (2) composed of 127 g of sodium acrylate, 36 g of acrylic acid, 0.077 g of polyethylene glycol diacrylate (n=9), 0.008 g of trisodium diethylenetriamine pentaacetate, and 215 g of ion-exchanged water, and nitrogen gas was blown into the aqueous monomer solution (2) to expel dissolved oxygen dissolved in the aqueous monomer solution (2). Next, after 1.5 g of a 15% aqueous solution of sodium persulfate had been added to the aqueous monomer solution in this flask, the whole amount of a resulting solution was added to the separable flask. A resulting mixture solution was stirred at 230 rpm for dispersion. After that, the temperature of a bath was increased to 60°C to initiate a polymerization reaction. After this bath temperature of 60°C was maintained for 2 hours, the polymerization was stopped, filtration was carried out by suction filtration, and a residue was air-dried overnight to obtain a hydrogel polymer (2). The average primary particle diameter of the hydrogel polymer (2) was 55 μm.

Next, the hydrogel polymer (2) (gel temperature: 90°C) was charged into a gel sizing apparatus having a screw and a porous plate having a hole diameter of 0.8 mm, and the hydrogel polymer (2) was discharged from the gel sizing apparatus to obtain a sized gel (2).

The sized gel (2) thus obtained was dried with use of a hot-air dryer to obtain a dried polymer (2). The drying was carried out by spreading, onto a stainless steel metal gauze having a mesh size of 850 pm, the sized gel (2) and allowing hot air of 105°C to pass through for 40 minutes.

The obtained dried polymer (2) was added to methanol (in an amount 10 times the weight of the dried polymer (2)) heated to 60°C and was stirred for 1 hour, followed by filtration and drying to carry out hydrophilization treatment.

Subsequently, the dried polymer (2) subjected to hydrophilization treatment was supplied to a roll mill (pulverizer) and pulverized to adjust the particle size, and further classified with use of a sieve having a mesh size particle diameter of 150 pm to obtain water-absorbing resin powder (2). The average particle diameter of the water-absorbing resin powder (2) was 310 pm.

To 100 parts by mass of the water-absorbing resin powder (2), a surface-crosslinking agent solution composed of 0.003 parts by mass of ethylene glycol diglycidyl ether, 0.385 parts by mass of ethylene carbonate, 0.644 parts by mass of propylene glycol, and 2.6 parts by mass of ion-exchanged water was sprayed with a spray, followed by uniform mixing with use of a high speed continuous mixer.

A resulting mixture was introduced into a heat treatment machine adjusted to an atmospheric temperature of

195°C±2°C and heat-treated for 25 minutes. Then, the powder temperature was forcibly cooled to 60°C to obtain surface-crosslinked water-absorbing resin powder (2).

To 100 parts by mass of the surface-crosslinked water-absorbing resin powder (2), 10.0 parts by mass of water was mixed uniformly. A resulting mixture was sized by passage through a JIS standard sieve having a mesh size of 1000 pm to obtain water-absorbing resin particles (2).

To 100 parts by mass of the obtained water-absorbing resin particles (2), 0.3 parts by mass of hydrotalcite (product name: DHT-6; available from Kyowa Chemical Industry Co., Ltd.; $Mg_6Al_2(OH)_{16}CO_3 \cdot 4H_2O$) was added and mixed to obtain a water-absorbing resin (2). Tables 1 to 3 show physical properties of the obtained water-absorbing resin (2).

[Production Example 1]

A water-absorbing resin (3) was obtained in the same manner as in Reference Example 2 except that, to 100 parts by mass of the water-absorbing resin particles (2) in Reference Example 2, instead of hydrotalcite, 0.3 parts by mass of silicon dioxide (trade name: Aerosil (registered trademark) 200; available from Nippon Aerosil Co., Ltd.) in the form of fine particles was added and mixed. Tables 1 to 3 show physical properties of the obtained water-absorbing resin (3).

[Production Example 2]

800 g of n-heptane was placed in a 2000-mL four-necked separable flask equipped with a stirrer, a reflux condenser, a thermometer, a nitrogen gas introduction tube, and a dropping funnel, 3.0 g of sucrose fatty acid ester (S-370; available from Mitsubishi Chemical Corporation; HLB=3) as a dispersing agent was added and dissolved, and nitrogen gas was blown into a resulting solution to expel dissolved oxygen.

Separately from this, prepared in a flask was an aqueous monomer solution (3) composed of 141 g of sodium acrylate, 36 g of acrylic acid, 0.209 g of polyethylene glycol diacrylate (n=9), and 214 g of ion-exchanged water, and nitrogen gas was blown into the aqueous monomer solution (3) to expel dissolved oxygen dissolved in the aqueous monomer solution (3). Next, after 2.0 g of a 10% aqueous solution of potassium persulfate had been added to the monomer solution in this flask, the whole amount of a resulting solution was added to the separable flask. A resulting mixture solution was stirred at 230 rpm for dispersion. After that, the temperature of a bath was increased to 85°C to initiate a polymerization reaction. After this bath temperature of 85°C was maintained for 2 hours, the polymerization was stopped, filtration was carried out by suction filtration, and a residue was air-dried overnight to obtain a hydrogel polymer (3). The average primary particle diameter of the hydrogel polymer (3) was 200 μm.

Next, the hydrogel polymer (3) (gel temperature: 90°C) was charged into a gel sizing apparatus having a screw and a porous plate having a hole diameter of 0.8 mm, and the hydrogel polymer (3) was discharged from the gel sizing apparatus to obtain a sized gel (3).

The sized gel (3) thus obtained was dried with use of a hot-air dryer to obtain a dried polymer (3). The drying was carried out by spreading, onto a stainless steel metal gauze having a mesh size of 850 pm, the sized gel (3) and allowing hot air of 180°C to pass through for 50 minutes.

200 g of the obtained dried polymer (3) was added to 10 L of methanol heated to 60°C and was stirred for 1 hour, followed by filtration and drying to carry out hydrophilization treatment.

Subsequently, the dried polymer (3) subjected to hydrophilization treatment was supplied to a roll mill (pulverizer) and pulverized to adjust the particle size, and further classified with use of a sieve having a mesh size particle diameter of 150 pm to obtain water-absorbing resin powder (3). The average particle diameter of the water-absorbing resin powder (3) was 350 pm.

To 100 parts by mass of the water-absorbing resin powder (3), a surface-crosslinking agent solution composed of 0.015 parts by mass of ethylene glycol diglycidyl ether, 1.0 part by mass of propylene glycol, and 3.0 parts by mass of ion-exchanged water was sprayed with a spray, followed by uniform mixing with use of a high speed continuous mixer.

A resulting mixture was introduced into a heat treatment machine adjusted to an atmospheric temperature of 195°C±2°C and heat-treated for 40 minutes. Then, the powder temperature was forcibly cooled to 60°C to obtain surface-crosslinked water-absorbing resin powder (3).

To 100 parts by mass of the surface-crosslinked water-absorbing resin powder (3), 10.0 parts by mass of water was mixed uniformly. A resulting mixture was sized by passage through a JIS standard sieve having a mesh size of 1000 pm to obtain water-absorbing resin particles (3).

To 100 parts by mass of the obtained water-absorbing resin particles (3), 0.1 parts by mass of silicon dioxide (trade name: Aerosil (registered trademark) 200; available from Nippon Aerosil Co., Ltd.) in the form of fine particles was added and mixed to obtain a water-absorbing resin (4). Tables 1 to 3 show physical properties of the obtained water-absorbing resin (4).

[Reference Example 3]

A comparative water-absorbing resin (1) was obtained in the same manner as in Reference Example 1, except that, in Reference Example 1, a hydrophilization treatment step was not carried out, and silicon dioxide in the form of fine particles was not used. Tables 1 to 3 show physical properties of the obtained comparative water-absorbing resin (1).

[Production Example 3]

500 g of n-heptane was placed in a 2000-mL four-necked separable flask equipped with a stirrer, a reflux condenser, a thermometer, a nitrogen gas introduction tube, and a dropping funnel, 0.92 g of sucrose fatty acid ester S-370 as a dispersing agent was added. The temperature was increased, and the dispersing agent was dissolved. After that, a resulting mixture solution was cooled to 55°C.

Separately from the above, 92 g of an 80 weight% aqueous acrylic acid solution was added to a 500-mL Erlenmeyer flask. To this, while being cooled from the outside, 102.2 g of a 30 weight% aqueous sodium hydroxide solution was added dropwise to neutralize 75 mol% of acrylic acid, so that an aqueous solution of partially neutralized acrylic acid was prepared. Further, 50.2 g of water, 0.11 g of potassium persulfate which served as a polymerization initiator, and 18.4 mg of ethylene glycol diglycidyl ether which served as a crosslinking agent were added to prepare an aqueous monomer solution for polymerization.

While being stirred, the whole amount of this aqueous monomer solution for polymerization was added for dispersion to the above-described four-necked separable flask. The inside of the system was sufficiently replaced with nitrogen, and then the temperature was increased. The bath temperature was maintained at 70°C, and the polymerization reaction was carried out for 1 hour. To a hydrogel-like substance obtained after the end of the polymerization, 0.66 g of a 14 mass% aqueous tetrasodium trans-1,2-diaminocyclohexane tetraacetate solution as an aminocarboxylic acid-based metal chelating agent was added while being stirred. After that, water was removed from the hydrogel-like substance to the outside of the system by azeotropic dehydration. To the obtained gel-like substance, 4.14 g of a 2 mass% aqueous ethylene glycol diglycidyl ether solution was added. Further, water and n-heptane were removed by distillation, followed by drying to obtain a comparative water-absorbing resin (2). Tables 1 to 3 show physical properties of the obtained comparative water-absorbing resin (2).

[Reference Example 4]

A water-absorbing resin was obtained by aqueous solution polymerization. That is, an aqueous monomer solution was prepared by mixing 67.0 parts of a 37% aqueous sodium acrylate solution, 10.2 parts of acrylic acid, 0.079 parts of polyethyleneglycol diacrylate (average number of ethylene oxide units: 8), and 22.0 parts of ion-exchanged water. Nitrogen was blown into the aqueous monomer solution in a vat to reduce the dissolved oxygen in the solution to 0.1 ppm or less.

Subsequently, the temperature of the aqueous monomer solution was adjusted to 18°C in a nitrogen atmosphere, and then 0.16 parts of a 5 mass% aqueous sodium persulfate solution, 0.16 parts of a 5% aqueous 2,2'-azobis(2-amidinopropane) dihydrochloride solution, 0.15 parts of a 0.5% aqueous L-ascorbic acid solution, and 0.17 parts of a 0.35% aqueous hydrogen peroxide solution were sequentially added dropwise while being stirred.

Polymerization was initiated immediately after the dropwise addition of hydrogen peroxide. After that, stirring was stopped, and after 10 minutes, the temperature of the monomer reached the peak temperature. The peak temperature was 85°C. Subsequently, the vat was immersed in a hot water bath of 80°C, followed by aging for 10 minutes to obtain a comparative hydrogel polymer (3).

The obtained comparative hydrogel polymer (3) was crushed with a meat chopper and then dried with use of a hot-air dryer to obtain a comparative dried polymer (3). The drying was carried out by spreading, onto a stainless steel metal gauze having a mesh size of 850 μm, the comparative hydrogel polymer (3) crushed with the meat chopper and allowing hot air of 180°C to pass through for 30 minutes.

The comparative dried polymer (3) was supplied to a roll mill (pulverizer) and pulverized to adjust the particle size, and further passed through a sieve having a mesh size particle diameter of 500 μm, and classified into particles remaining on a 105-pm sieve to obtain comparative water-absorbing resin powder (3). The average particle diameter of the comparative water-absorbing resin powder (3) was 370 pm.

To 100 parts by mass of the comparative water-absorbing resin powder (3), a surface-crosslinking agent solution composed of 0.05 parts by mass of ethylene glycol diglycidyl ether, 1 part by mass of propylene glycol, 3 parts by mass of ion-exchanged water, and 1 part by mass of isopropyl alcohol was sprayed with a spray, followed by uniform mixing with use of a high speed continuous mixer. A resulting mixture was introduced into a heat treatment machine adjusted to an atmospheric temperature of 180°C±2°C and heat-treated for 40 minutes. Then, the powder temperature was forcibly cooled to 60°C to obtain surface-crosslinked comparative water-absorbing resin powder (3).

To 100 parts by mass of the surface-crosslinked comparative water-absorbing resin powder (3), a solution composed of 0.022 parts by mass of 45 mass% trisodium salt of diethylenetriamine pentaacetate, and 1 part by mass of ion-exchanged water was mixed uniformly to obtain comparative water-absorbing resin particles (3).

To 100 parts by mass of the obtained comparative water-absorbing resin particles (3), 0.5 parts by mass of silicon dioxide (trade name: Aerosil (registered trademark) 200; available from Nippon Aerosil Co., Ltd.) in the form of fine particles was added and mixed to obtain a comparative water-absorbing resin (3). Tables 1 to 3 show physical properties of the obtained comparative water-absorbing resin (3).

[Table 1]

| Physical properties of water-absorbing resins | | | | | | | | |
|---|---|---|---|---|---|---|---|---|
| | | CRC [g/g] | AAP [g/g] | Ext [%] | Moisture content [%] | D50 [μm] | Bulk density [g/mL] | Surface tension [mN/m] |
| Reference Example 1 | Water-absorbing resin (1) | 34.0 | 23.0 | 22.0 | 14.0 | 80 | 0.83 | 72 |
| Reference Example 2 | Water-absorbing resin (2) | 32.3 | 19.9 | 19.5 | 12.1 | 320 | 0.68 | 71 |
| Production Example 1 | Water-absorbing resin (3) | 32.4 | 20.8 | 19.1 | 11.9 | 320 | 0.71 | 70 |
| Production Example 2 | Water-absorbing resin (4) | 38.8 | 12.8 | 26.6 | 11.3 | 360 | 0.70 | 71 |
| Reference Example 3 | Comparative water-absorbing resin (1) | 33.8 | 23.6 | 21.9 | 14.1 | 80 | 0.83 | 66 |
| Production Example 3 | Comparative water-absorbing resin (2) | 33.3 | 14.8 | 22.2 | 12.5 | 110 | 0.91 | 61 |
| Reference Example 4 | Comparative water-absorbing resin (3) | 34.0 | 19.0 | 16.0 | 1.0 | 380 | 0.64 | 73 |

[Table 2]

| Liquid diffusion lengths of water-absorbing resins | | | | |
|---|---|---|---|---|
| | | Liquid diffusion length D [mm] | Liquid diffusion length L [mm] | L/D |
| Reference Example 1 | Water-absorbing resin (1) | 23 | 20 | 0.87 |
| Reference Example 2 | Water-absorbing resin (2) | 17 | 23 | 1.35 |
| Production Example 1 | Water-absorbing resin (3) | 19 | 20 | 1.05 |
| Production Exam ple 2 | Water-absorbing resin (4) | 18 | 24 | 1.33 |
| Reference Example 3 | Comparative water-absorbing resin (1) | 12 | 38 | 3.17 |
| Production Example 3 | Comparative water-absorbing resin (2) | 16 | 33 | 2.06 |
| Reference Example 4 | Comparative water-absorbing resin (3) | 15 | 30 | 2.00 |

[Table 3]

| Evaluation results for absorbent sheets for evaluation | | | |
|---|---|---|---|
| | | Evaluation of absorbent sheet Amount of return [g] | State of absorption after liquid injection |
| Reference Example 1 | Water-absorbing resin (1) | 0.20 | Quickly absorbed |
| Reference Example 2 | Water-absorbing resin (2) | 0.29 | Quickly absorbed |
| Production Example 1 | Water-absorbing resin (3) | 0.01 | Quickly absorbed |
| Production Example 2 | Water-absorbing resin (4) | 0.01 | Quickly absorbed |
| Reference Example 3 | Comparative water-absorbing resin (1) | 1.90 | Slow to become compatible with liquid |
| Production Example 3 | Comparative water-absorbing resin (2) | 3.54 | Slow to become compatible with liquid |
| Reference Example 4 | Comparative water-absorbing resin (3) | 2.45 | Liquid remaining between gels present |

From the description in Table 2, it was found that the water-absorbing resins (3) and (4) respectively prepared in Production Examples 1 and 2 each correspond to the water-absorbing resin in the present invention. In contrast, it was found that the comparative water-absorbing resin (2) prepared in Production Example 3 does not correspond to the water-absorbing resin in the present invention.

[Production of absorbent article]

[Example 1]

A sheet obtained by cutting a desalted vinyl tape available from Nitto Denko Corporation to dimensions of 10 cm in width by 18 cm in length was used as a liquid-impermeable back sheet. A sheet obtained by cutting a polypropylene nonwoven fabric (spunbond nonwoven fabric; basis weight: 13 g/$m^2$) to dimensions of 10 cm in width by 18 cm in length was used as a liquid-permeable top sheet. Pulverized pulp, which is a hydrophilic fiber material, was uniformly placed in a rectangular portion of 8 cm in width and 16 cm in length which is provided 1 cm inward of an edge on each of the four sides of the liquid-impermeable back sheet. Next, 5.12 g (basis weight of 400 g/$m^2$) of the water-absorbing resin (4) was uniformly sprayed over the pulverized pulp. Further, from above the water-absorbing resin (4), 0.5 g of pulverized pulp, which is a hydrophilic fiber material, was uniformly sprayed over, and a weight of 1 kg having a bottom surface of 8 cm in width and 16 cm in length was placed in an area where the pulverized pulp and the water-absorbing resin (4) were placed to pressurize for 1 minute. Thereafter, the weight was removed. The liquid-permeable top sheet was overlaid on the area where the pulverized pulp and the water-absorbing resin (4) were placed to sandwich the pulverized pulp and the water-absorbing resin (4), and respective portions of the sheets which are provided 1.0 cm inward of an edge on each of the four sides were bonded to produce an absorbent article. The produced absorbent article was regarded as the absorbent article (1).

[Example 2]

An absorbent article was produced in the same method as in Example 1, except that the water-absorbing resin (3) prepared in Production Example 3 was used instead of the water-absorbing resin (4). The produced absorbent article was regarded as the absorbent article (2).

[Comparative Example 1]

An absorbent article was produced in the same method as in Example 1, except that the comparative water-absorbing resin (2) prepared in Production Example 3 was used instead of the water-absorbing resin (4). The produced

absorbent article was regarded as the comparative absorbent article (1).

[Comparative Example 2]

An absorbent article was produced in the same method as in Example 1, except that the basis weight of the water-absorbing resin in the absorbent body, that is, the basis weight of the water-absorbing resin in a resulting absorbent article, was changed to 200 g/$m^2$ by changing the amount of the water-absorbing resin (4) used to 2.56 g. The produced absorbent article was regarded as the comparative absorbent article (2).

[Results]

For the absorbent articles (1) and (2) respectively produced in Examples 1 and 2 and the comparative absorbent articles (1) and (2) respectively produced in Comparative Examples 1 and 2, the liquid diffusion lengths in the longitudinal, lateral, upward, and downward directions were measured by the aforementioned methods. The results of the measurements are shown in Table 4. In addition, a ratio between the liquid diffusion length in the lateral direction and the liquid diffusion length in the longitudinal direction, a ratio between the liquid diffusion length in the upward direction and the liquid diffusion length in the downward direction, and a value obtained by division of the liquid diffusion length in the lateral direction by 2 were calculated. The results of the calculations are also shown in Table 4 below.

[Table 4]

| Evaluation results for absorbent articles | | | | | | | | | |
|---|---|---|---|---|---|---|---|---|---|
| | Type of water-absorbing resin | Basis weight of water-absorbing resin | Liquid diffusion length in lateral direction | Liquid diffusion length in longitudinal direction | Liquid diffusion length in upward direction from liquid charging portion | Liquid diffusion length in downward direction from liquid charging portion | Ratio between liquid diffusion lengths (lateral direction/ longitudinal direction) | Ratio between liquid diffusion lengths from liquid charging portion (upward direction/ downward direction) | Liquid diffusion length in lateral direction/ 2 |
| | | g/m2 | cm | cm | cm | cm | - | - | cm |
| Example 1 | Water-absorbing resin (4) | 400 | 6.0 | 5.9 | 2.6 | 3.3 | 1.02 | 0.8 | 3.0 |
| Example 2 | Water-absorbing resin (3) | 400 | 5.8 | 4.9 | 2.2 | 2.7 | 1.18 | 0.8 | 2.9 |
| Comparative Example 1 | Comparative water-absorbing resin (2) | 400 | 8.2 | 8.8 | 2.5 | 6.3 | 0.93 | 0.4 | 4.1 |
| Comparative Example 2 | Water-absorbing resin (4) | 200 | 7.5 | 4.3 | 1.8 | 2.5 | 1.74 | 0.7 | 3.8 |

As shown in Table 4, the absorbent articles produced in Examples 1 and 2, each of which includes a water-absorbing resin corresponding to the water-absorbing resin in the present invention, have a shorter liquid diffusion length in the lateral direction and have less liquid running in the direction along the crease of the absorbent article than the absorbent article produced in Comparative Example 1, which includes a water-absorbing resin that does not correspond to the water-absorbing resin in the present invention. Therefore, it was found that, thanks to containing the water-absorbing resin in the present invention, the absorbent article in accordance with an embodiment of the present invention, in a case where the absorbent article is allowed to absorb a water-based liquid in a state in which the absorbent article has a crease, reduces or prevents the occurrence of liquid running along the crease. Further, thanks to containing the water-absorbing resin in the present invention, the absorbent article in accordance with an embodiment of the present invention exhibits the same diffusion lengths in the downward direction and in the upward direction from the crease and thus enables the whole absorbent body to absorb the water-based liquid. This achieves good efficiency.

The absorbent article produced in Example 1, in which the basis weight of the water-absorbing resin is in the range of 300 $g/m^2$ to 1000 $g/m^2$, has a shorter liquid diffusion length in the lateral direction and has less liquid running in the direction along the crease of the absorbent article than the absorbent article produced in Comparative Example 2, in which the same water-absorbing resin as that used in Example 1 is contained, but the basis weight of the water-absorbing resin is less than 300 $g/m^2$ and falls outside the above-described range. Therefore, it was found that, thanks to having a basis weight of the water-absorbing resin in the range of 300 $g/m^2$ to 1000 $g/m^2$, the absorbent article in accordance with an embodiment of the present invention, in a case where the absorbent article is allowed to absorb a water-based liquid in a state in which the absorbent article has a crease, reduces or prevents the occurrence of liquid running along the crease.

From the above facts, it was found that, thanks to containing the water-absorbing resin in the present invention and having the basis weight of the water-absorbing resin in the range of 300 $g/m^2$ to 1000 $g/m^2$, the absorbent article in accordance with an embodiment of the present invention, in a case where the absorbent article is allowed to absorb a water-based liquid in a state in which the absorbent article has a crease, reduces or prevents the occurrence of liquid running along the crease and consequently brings about the effect of making it possible to reduce or prevent leakage of the water-based liquid from sides of the absorbent article due to the occurrence of the liquid running.

Industrial Applicability

An absorbent article in accordance with an embodiment of the present invention can be suitably used in various applications such as hygienic materials (sanitary materials) including disposable diapers, sanitary napkins, incontinence articles for adults (incontinence pads), and sheets for pets, water retaining agents for soil for agricultural/horticultural use, and industrial waterproofing agents, and the like.

Reference Signs List

1: water-absorbing resin
2: container
3: colored region
4: absorbent sheet for evaluation
5: non-woven fabric
6: adhesive tape
7: absorbent article
8: liquid feed tube
9: tilt stand

## Claims

1. An absorbent article comprising a liquid-permeable top sheet, an absorbent body containing a water-absorbing resin, and a liquid-impermeable back sheet in this order, wherein

   a basis weight of the water-absorbing resin is 300 $g/m^2$ to 1000 $g/m^2$, and
   the water-absorbing resin is a particulate poly(meth)acrylic acid (salt)-based water-absorbing resin and has the following physical property values (1) to (3):

(1) a vertical liquid diffusion length (D) is 15 mm or more;
(2) a ratio (L/D) of a horizontal liquid diffusion length (L) to the vertical liquid diffusion length is 0.5 to 1.5; and
(3) a bulk density defined in EDANA ERT 460.2-02 is 0.68 g/mL or more,

where the vertical liquid diffusion length (D) and the horizontal liquid diffusion length (L) are determined by filling a transparent container made of an acrylic resin (having internal dimensions of 40 mm in a vertical direction (height) by 72 mm in a horizontal direction (width) by 3 mm in depth) with the water-absorbing resin up to a position at a height of 30 mm, injecting, from a surface of the water-absorbing resin, 1 mL of a 0.9 mass% aqueous sodium chloride solution which is colored and has a temperature of 20°C, and measuring a diffusion length in the vertical direction and a diffusion length in the horizontal direction 1 minute after the injection.

**2.** The absorbent article according to claim 1, wherein the water-absorbing resin is an aggregate-like particle of spherical particles.

**3.** The absorbent article according to claim 1 or 2, wherein the water-absorbing resin includes water-insoluble inorganic fine particles.

**4.** The absorbent article according to any one of claims 1 to 3, wherein the water-absorbing resin has a mass average particle diameter (D50) of 200 pm to 700 pm.

**5.** The absorbent article according to any one of claims 1 to 4, wherein the water-absorbing resin has an AAP of 18 g/g or more.

**6.** The absorbent article according to any one of claims 1 to 5, wherein the water-absorbing resin has a bulk density defined in EDANA ERT 460.2-02 of 0.70 g/mL or more.

**7.** The absorbent article according to any one of claims 1 to 6, wherein the absorbent body further contains a hydrophilic fiber material, and a mass of the water-absorbing resin accounts for 50 mass% or more and less than 100 mass% of a total mass of the water-absorbing resin and the hydrophilic fiber material.

**8.** The absorbent article according to any one of claims 1 to 7, wherein said absorbent article is packaged in a folded state.

FIG. 1

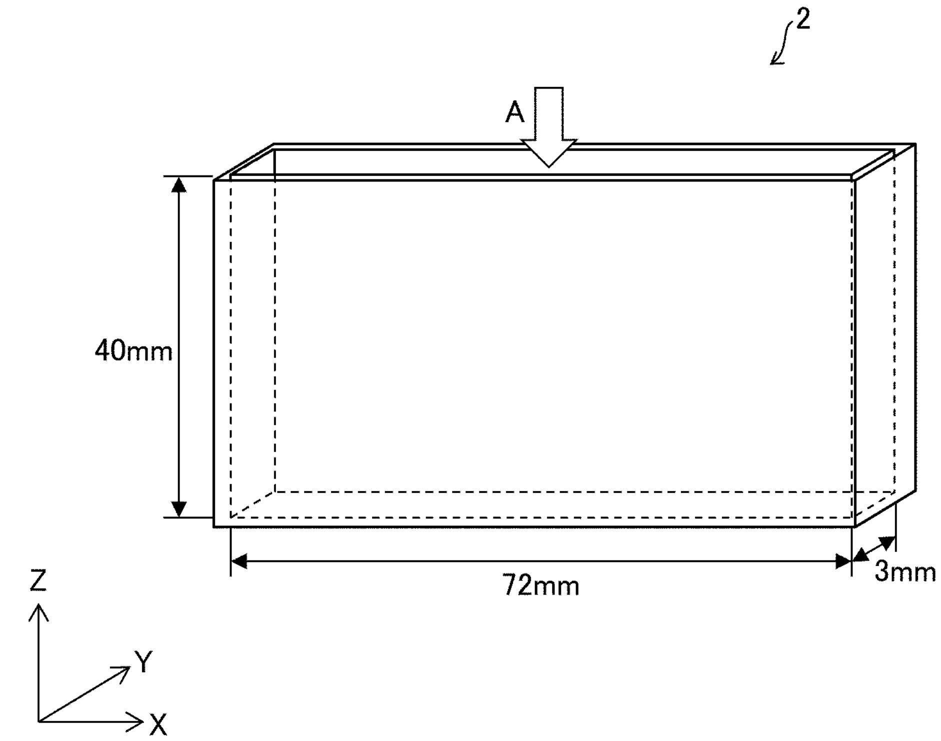


FIG. 2

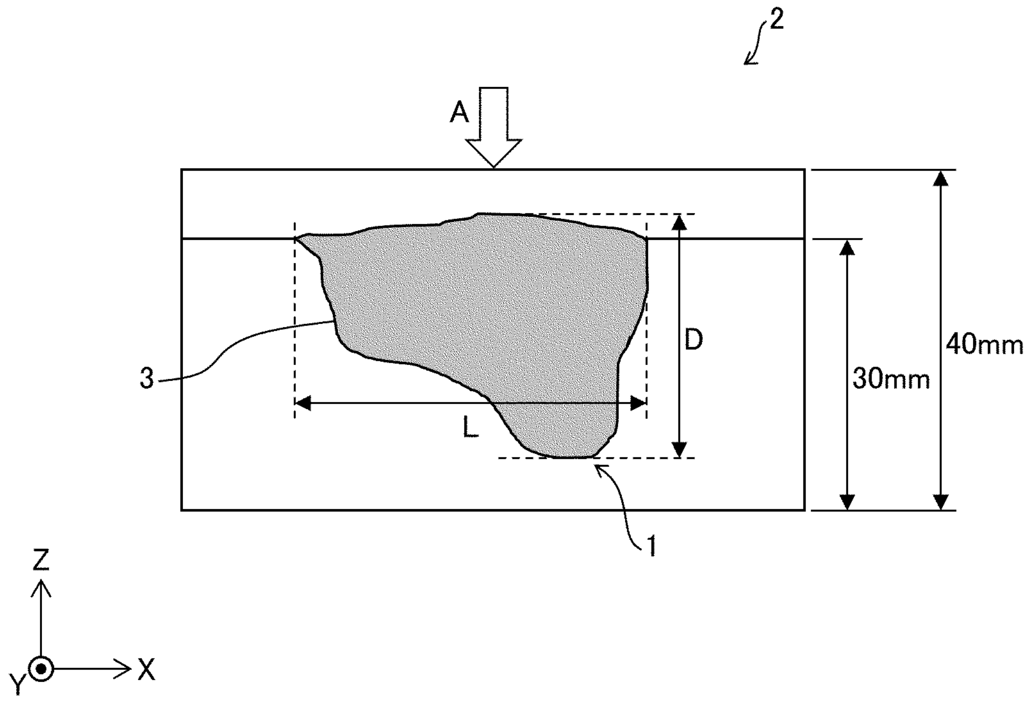

FIG. 3

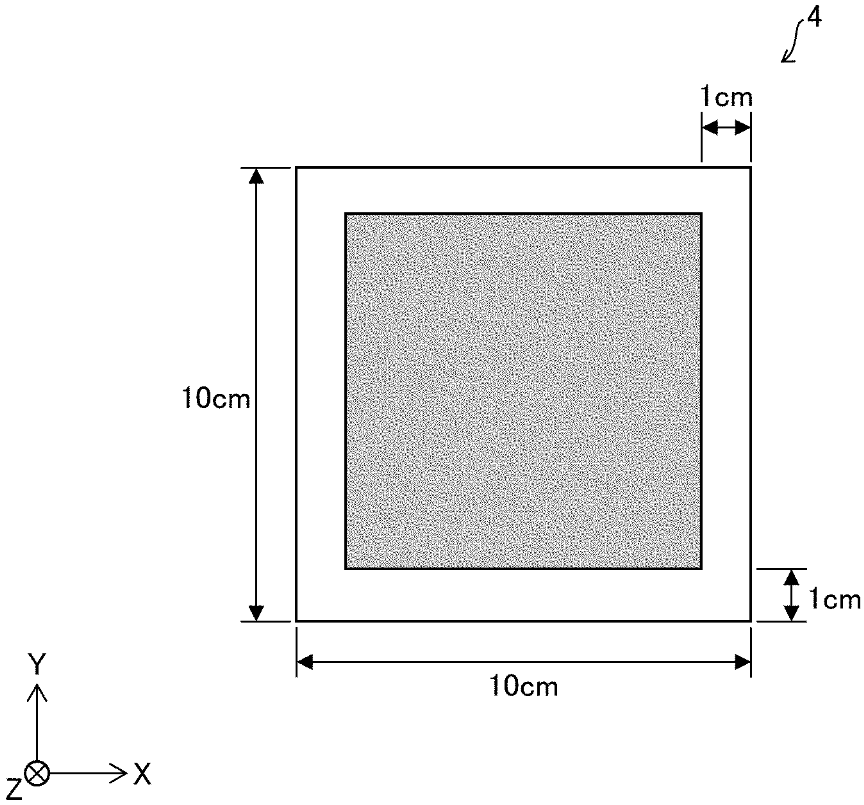


FIG. 4

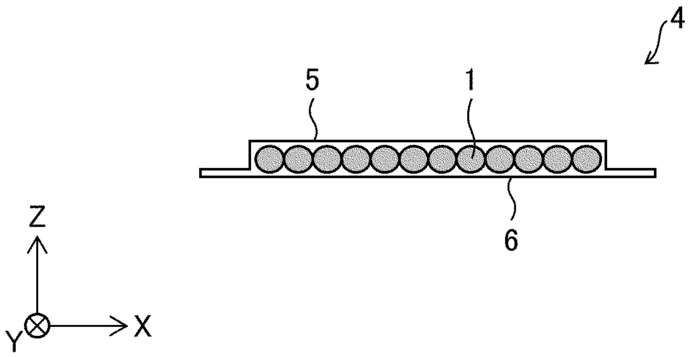

FIG. 5

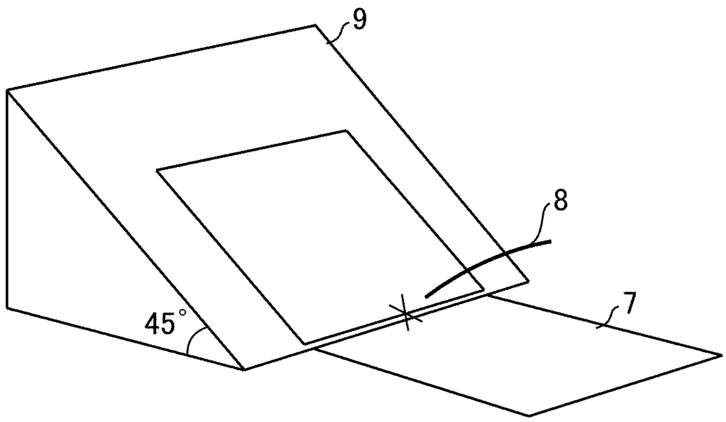
9
8
7
45°

FIG. 6

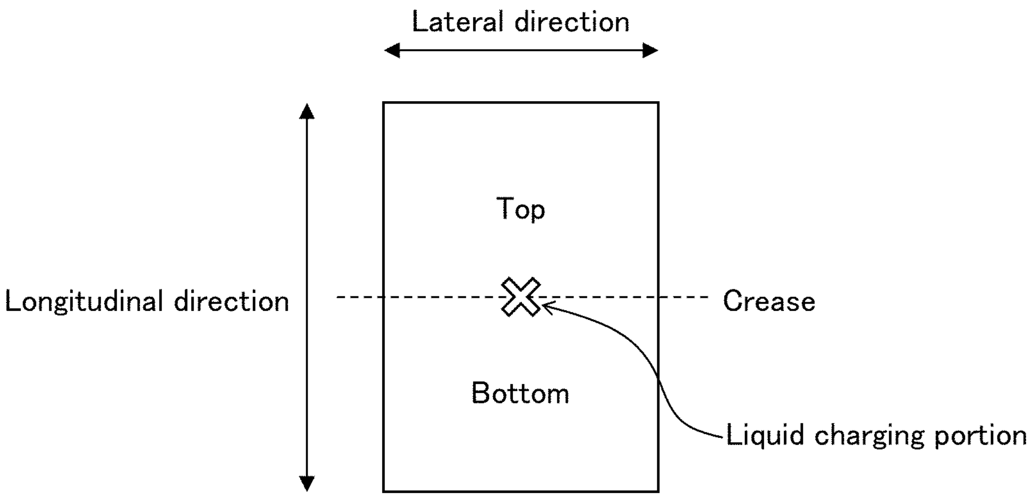
Lateral direction
Top
Longitudinal direction
Crease
Bottom
Liquid charging portion

# INTERNATIONAL SEARCH REPORT

International application No.
**PCT/JP2022/044391**

## A. CLASSIFICATION OF SUBJECT MATTER

***C08J 3/16***(2006.01)i; ***C08J 3/24***(2006.01)i; ***A61F 13/53***(2006.01)i; ***A61F 13/537***(2006.01)i; ***B01J 20/26***(2006.01)i; ***B01J 20/28***(2006.01)i
FI: A61F13/53 300; B01J20/26 D; B01J20/28 Z; C08J3/16; A61F13/537 400; C08J3/24 Z CEY

According to International Patent Classification (IPC) or to both national classification and IPC

## B. FIELDS SEARCHED

Minimum documentation searched (classification system followed by classification symbols)

C08J3/16; C08J3/24; A61F13/53; A61F13/537; B01J20/26; B01J20/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| Y | WO 2019/098244 A1 (NIPPON SHOKUBAI CO., LTD.) 23 May 2019 (2019-05-23) paragraphs [0023], [0024], [0059], [0096], [0139], [0216], [0348], [0637], [0638] | 1-8 |
| Y | JP 2016-7340 A (NIPPON PAPER CRECIA CO., LTD.) 18 January 2016 (2016-01-18) paragraph [0021] | 1-8 |
| Y | JP 2016-536078 A (BASF SE) 24 November 2016 (2016-11-24) paragraphs [0262], [0263], [0537] | 1-8 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 January 2023** | **31 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/044391**

| Patent document cited in search report | Publication date (day/month/year) | Patent family member(s) | Publication date (day/month/year) |
|---|---|---|---|
| WO 2019/098244 A1 | 23 May 2019 | US 2020/0398253 A1<br>paragraphs [0061]-[0064], [0119], [0174], [0227], [0328], [0496], [0940], [0941]<br>EP 3711722 A1<br>CN 111356426 A<br>KR 10-2020-0087197 A | |
| JP 2016-7340 A | 18 January 2016 | (Family: none) | |
| JP 2016-536078 A | 24 November 2016 | US 2015/0299404 A1<br>paragraphs [0340], [0341], [0379]<br>WO 2015/028327 A1<br>EP 3381956 A1<br>KR 10-2015-0087368 A<br>CN 104936997 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11071425 A
- JP 02918807 B
- WO 2020122215 A
- WO 2021049450 A
- WO 2011040530 A
- EP 0629411 A
- JP 6837139 B
- US 7638570 B
- WO 2015129917 A
- WO 2009025235 A
- WO 2013018571 A
- WO 2015053372 A